(19) Europäisches Patentamt
European Patent Office
Office européen des brevets

(11) Publication number: **0 173 629 B1**

(12) **EUROPEAN PATENT SPECIFICATION**

(45) Date of publication of patent specification: **10.06.92**

(51) Int. Cl.[5]: **G01N 33/534**, G01N 33/577, A61K 49/02, A61K 43/00, A61K 39/395

(21) Application number: **85401695.3**

(22) Date of filing: **29.08.85**

The file contains technical information submitted after the application was filed and not included in this specification

(54) **Antibody-metal ion complexes.**

(30) Priority: **31.08.84 US 646327**
**31.08.84 US 646328**

(43) Date of publication of application:
**05.03.86 Bulletin 86/10**

(45) Publication of the grant of the patent:
**10.06.92 Bulletin 92/24**

(84) Designated Contracting States:
**AT BE CH DE FR GB IT LI LU NL SE**

(56) References cited:
**DE-A- 3 239 410**

**Cancer Drug Delivery, vol. 1, no. 2, 1984, J. Powe et al. "Labeling Monoclonal Antibodies and F(ab')2 Fragments with (111In) Indium using cyclic DTPA Anhydride and Their In Vivo Behavior in Mice Bearing Human Tumor Xenografts", pages 125-135**

**Science, vol. 220, no. 4597, May 6, 1983, D.J. Hnatowich et al. "Radioactive Labeling of Antibody: A Simple and Efficient Method", pages 613-615**

(73) Proprietor: **CYTOGEN CORPORATION**
**201 College Road East Forrestal Research Center**
**Princeton New Jersey 08540(US)**

(72) Inventor: **Lee, Chyi**
**1347 Ute Road**
**New Brunswick New Jersey(US)**
Inventor: **Rodwell, John Dennis**
**430 Ramsey Road**
**Yardley Pennsylvania(US)**
Inventor: **Goers, John Walter Frank**
**5200 Dolores Street**
**Atascaders California(US)**
Inventor: **Siegel, Richard Charles**
**388 Colonel Greene Road**
**Yorktown Heights New York(US)**
Inventor: **Alvarez, Vernon Leon**
**187 Rice Drive**
**Morrisville Pennsylvania(US)**
Inventor: **McKearn, Thomas Joseph**
**RD 3, Box 119**
**New Hope Pennsylvania(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

**Cancer Treatment Reports, vol. 68, no. 1, January 1984, Jorge A. Carrasquillo et al. "Diagnosis of and Therapy for Solid Tumors With Radiolabeled Antibodies and Immune Fragments", pages 317-328**

(74) Representative: **Ahner, Francis et al**
**CABINET REGIMBEAU, 26, avenue Kléber**
**F-75116 Paris(FR)**

**Description**

1. FIELD OF THE INVENTION

The present invention relates to the general area of antibody systems capable of delivering metal ions to target sites in vivo or in vitro. For embodiments in which the metal ions are radioisotopes or another detectible ion, the antibody systems are capable of being identified and/or detected owing to the radioisotope or other detectible ion attached to the antibody. More particularly, the invention is directed to water-soluble antibody-chelator-metal ion complexes comprising metal ions attached by coordinate bonding via a compatible chelator to an antibody molecule which substantially retains the immunospecificity and immunoreactivity of the original antibody. The invention also relates to several methods for preparing such antibody-chelator-metal ion complexes as well as antibody-chelator conjugates which are useful in preparing such complexes.

The antibody-chelator-metal ion complexes of the invention may he used in a variety of in vitro or in vivo applications involving detection or delivery of a labeled antibody or a metal ion, including, but not limited to, immunological assays, imaging of specific tissues and delivery of metal ions and/or radioisotopes to specific tissue sites.

2. BACKGROUND OF THE INVENTION

A number of agents have been utilized as carrier molecules with limited success in imaging systems. In practice the carrier should be non-toxic and target site specific. Ideally there should be a mechanism for maintenance of the detectable compound or agent at the target site.

Radiopharmaceutical techniques currently used in non-invasive in vivo imaging methods are based upon the ability of the target organ to remove the radiopharmaceutical label from the circulation. These techniques utilize various substances to deliver radioactive compounds to desired target; such substances include substrates, substrate analogs, ligands, hormones, radionuclides, bifunctional chelates (linker groups containing a chelator at one end which is able to bind a heavy metal or radioisotope and a reactive group at the other end which can covalently attach to a target cell) and liposomes (Eckelman and Levanson, 1977, Intl. J. Appl. Radiat. Isot. 28: 67-82).

Other non-invasive techniques currently available are emission tomography, nuclear magnetic resonance imaging, and in vitro spectroscopy. A method employing isothiocyanate as a coupling agent has been used to attach fluorescent compounds to antibody molecules for use in fluorescence microscopy (Brandtzaeg, 1973, Scand. J. Immunol. 2: 273-290).

2.1. RADIOIMMUNOIMAGING USING MONOCLONAL ANTIBODIES

Monoclonal antibodies produced by the hybridoma technique of Kohler and Milstein (1975, Nature 256: 495-497; 1976, Eur. J. Immunol. 6: 511-519) or related techniques provide distinct advantages for use as carriers for imaging systems. First, monoclonal antibodies bind to only one molecular site (i.e., an epitope) with specific binding constants. Second, such antibodies are homogeneous and thus are purified with relative ease. Third, monoclonal antibodies can be made in large quantities by particular hybridoma cell lines.

The discovery of tumor-produced or tumor-associated antigens has allowed the preparation of monoclonal antibodies which are immune-specific for solid tumors such as human colon, breast, hepatoma, melanoma and germ cell tumors (see reviews by Carrasquillo, et al., 1984, Cancer Treatment Repts. 68: 317-328; Kennel, et al., 1984, Bio. Sci. 34: 150-156). Methods have been developed for detecting and locating tumors utilizing antibodies or antibody fragments specific for a variety of categories of tumor-associated antigens, including: oncofetal antigens, placental antigens, oncogenic or tumor virus-associated antigens, tissue-associated antigens, organ-associated antigens, ectopic hormones, normal antigens and variants thereof. For example, Goldenberg, et al. have demonstrated clinical detection of tumors in human patients using 131I-radiolabeled antibody specific for carcinoembryonic antigen (1978, New Eng. J. Med. 298: 1384-1388; see also disclosures in United States Patent No. 4,331,647 issued to Goldenberg, United States Patent No. 3,927,193 to Hansen, et al.). The methods utilized by Goldenberg, et al., however, required either repeated injection of other radioactive materials or single injection of mixtures of radioactive antibody in order to attain sufficient resolution to distinguish tumors from non-target areas.

The development of monoclonal antibodies specific for myosin, a contractile proteinaceous substance that is accessible to circulating antibody upon cardiac cell death or damage, has allowed development of

methods utilizing radioimmunoimaging to locate and quantify myocardial infarcts (see United States Pat. 4,036,945 to Haber). Strauss (1984, Diagnostic Imaging Feb. 1984, p. 54) highlights possible uses and potential problems with such antibodies.

## 2.2. ATTACHMENT OF RADIOLABELS

A number of radioisotopes including $^{125}$Iodine, $^{131}$Iodine, $^{111}$Indium, and $^{67}$Copper have been attached to monoclonal or polyclonal antibodies for use in detection of tumors in vivo in animal models. Vincour (1984, Diagnostic Imaging, Feb. 1984, pp. 56-61) reviews the problems experienced with such radiolabeled antibodies, noting especially the need for development of radiolabels which remain more stably affixed to the antibody molecule (Id. at 57).

Recently Hnatowich, et al. (1983, Science 220 : 613-615) and Powe, et al. (1984, Cancer Drug Delivery 1 : 125-135) described a method of attaching $^{111}$In to antibody molecules or fragments utilizing the water-soluble cyclic or bicyclic anhydride of diethylenetriaminepentaacetic acid (DTPA). In both these references the antibody was attached to the water-soluble chelator, DTPA, via an acylation reaction.

The DE-A1-3239410 describes radiolabeling, either directly or via a chelate-bound radionucleide, of monoclonal antibodies or antibody fragments. Attachment of the chelating group to the antibody was obtained by coupling to reactive groups on the polypeptide moiety of the antibody. Such groups occur randomly throughout the polypeptide moiety of the antibody. As a consequence, the modification of the antibody is significantly less selective than the method of the present invention.

## 2.3. RADIOIMMUNOIMAGES AND LOCALIZATION

A common feature of prior art in vivo images generated either with radioactive metal chelates or $^{131}$Iodine attached to antibodies is non-targeted localization in organs such as liver or spleen (or thyroid for $^{131}$I). Such non-specific localization results in images which do not accurately delineate the position, orientation or size of the intended target. Frequently, "background subtraction" of non-specifically localized radioactivity does not adequately compensate.

## 2.4. RADIOIMMUNOTHERAPY

The primary distinctions between the materials used in radioimmunoimaging and radioimmunotherapy are the amount and type the radiation employed. In a therapeutic context, radiation is employed to kill target cells while causing minimal toxicity to non-targeted cells. Thus, the radiation ideally has high energy and a short travel range in tissue. Since therapeutic applications involve antibody conjugates which are lethal to cells, any non-specific localization presents a serious problem for which one cannot compensate by "background subtraction". Consequently, products and methods which provide limited success with in vivo imaging may not be suitable at all for therapeutic applications.

# 3. SUMMARY OF THE INVENTION

According to the general methods of the present invention, a metal ion is attached to an antibody or antibody fragment directed against a target antigen. Attachment is accomplished using chelators (i.e., compounds that are able to donate electrons and combine by coordinate bonding with a metal ion to form structures termed chelates or chelation complexes) between the metal ion and the antibody. After formation of antibody-chelator-metal ion complexes, non-specifically attached metal ions should be removed. According to the preferred embodiment of the present invention then, removal of non-specifically attached metal ions from the complexes is accomplished using a high performance liquid molecular sieve chromatography system. This enhances the precision and resolution of the radioimages obtained when using the complexes in vivo, and represents a significant improvement over prior methods in which less than 50% of the metal ion may be specifically bound to the chelator portion of the antibody-chelator conjugate, and in which some of the antibody may be aggregated.

In its most general concept, the invention contemplates site selective attachment of compatible chelators to those areas of antibodies or antibody fragments which are not a part of nor directly involved with the antigenic site of the molecule. Thus, after selective attachment of a compatible chelator to one of these sites (located outside the antigen binding region), the antibody conjugate formed has substantially the same immunoreactivity and immunospecificity as the unconjugated antibody or antibody fragment.

In the present invention, compatible chelators containing an amine group selected from the group

consisting of primary amine, hydrazine, hydrazide, hydroxylamine, phenylhydrazine, semicarbazide and thiosemicarbazide groups are attached directly to the oxidized carbohydrate moieties of the antibodies or antibody fragments according to methods of attachment described herein.

The present invention provides an antibody-chelator conjugate as claimed in any one of claims 1 to 6, and antibody chelator metal ion complexes as claimed in any one of claims 7-18 as well as a composition for therapeutic treatment of cellular disorder comprising an antibody chelator metal ion complex as claimed in claim 19 and a method for testing for antigen as claimed in claim 20.

## 4. BRIEF DESCRIPTION OF THE FIGURES

The invention may be more fully understood by reference to the appended drawings in which:

FIG. 1 schematically represents the elution pattern of $^{111}$Indium-CYT-015-p-aminoaniline-diethylenetriaminepentaacetic acid ($^{111}$In-CYT-015-ADTPA) ON high pressure liquid gel permeation chromatography (see Section 6.2.2).

FIG. 2 represents autoradiographic images of Brown Norway (BN) tumor-bearing nude mice injected with $^{111}$Indium-labeled anti-Brown Norway (BN) major histocompatibility complex (MHC) antibody-metal ion complex, $^{111}$In-CYT-015-ADTPA. Images A, B, C and D were taken 24, 24, 48 and 72 hours after injection, respectively (see Section 8.1).

FIG. 3 presents autoradiographic images of control BN tumor-bearing nude mice injected with $^{111}$Indium-labeled anti-human MHC antibody-metal ion complex, $^{111}$In-CYT-012-ADTPA. Images E and F were taken 24 and 72 hours after injection, respectively (see Section 8.1).

FIG. 4 represents an autoradiographic image of control non-tumor-bearing nude mice injected with 111Indium-labeled anti-BN MHC antibody-metal ion complex, $^{111}$In-CYT-015-ADTPA. Images G and H were taken 24 and 72 hours after injection, respectively (see Section 8.1).

FIG. 5 represents an autoradiographic image of a Lewis rat with a transplanted BN X Lewis rat kidney injected with $^{111}$Indium-labeled anti-BN antibody-metal ion complex, $^{111}$In-CYT-015-ADTPA (image A) and then $^{99m}$Technetium-DTPA (image B, see Section 8.2).

## 5. DETAILED DESCRIPTION OF THE INVENTION

The instant invention concerns antibody-metal ion complexes prepared by attaching a metal ion to an antibody or antibody fragment directed against a target antigen. Compatible chelators capable of coordinate bonding with a metal ion are utilized to attach the metal ions to the antibody or antibody fragment. Such chelators are selectively attached to those areas of antibodies or antibody fragments which are not a part of nor directly involved with the antigenic site of the molecule.

In particular, the invention concerns methods for preparing antibody-metal ion complexes, comprising:

(a) reacting an antibody or antibody fragment with an oxidizing agent to form an aldehyde group in the carbohydrate moiety of the antibody or antibody fragment;

(b) reacting the aldehyde group of the resultant oxidized antibody or antibody fragment with a compatible chelator containing an amine group selected from the group consisting of primary amine, hydrazine, hydrazide, hydroxylamine, phenylhydrazine, semicarbazide and thiosemicarbazide groups to form an antibody-chelator conjugate having substantially the same immunoreactivity and immunospecificity as the unconjugated antibody or antibody fragment; and

(c) combining the antibody-chelator conjugate with a metal ion under conditions which allow chelation of the metal ion to the chelator of the antibody-chelator conjugate to form an antibody-chelator-metal ion complex.

In certain circumstances, it may be desirable to separate the above-described method for preparing antibody-chelator-metal ion complexes into two parts. The first part would produce an antibody-chelator conjugate, which may be considered an intermediate in the production of the final antibody-chelator-metal ion complex. Such antibody-chelator conjugates may be stored for later combination with the particular metal ion of interest. Thus, the first part of the two part method would involve steps (a) and (b) above to form the intermediate antibody-chelator conjugate. The second part, possibly at a later point in time, would involve complexing the antibody-chelator conjugate with a metal ion to produce the final antibody-chelator-metal ion complex.

Such antibody-chelator-metal ion complexes can also be made by alternate methods, as, for example, by first coordinately bonding the compatible chelator to the metal ion, and then reacting the antibody or antibody fragment with the chelator-metal ion complex to form the antibody-chelator-metal ion complex. Thus, the invention further includes a method for preparing an antibody-chelator-metal ion complex,

comprising:

(a) reacting an antibody or antibody fragment with an oxidizing agent to form an aldehyde group in the carbohydrate moiety of the antibody or antibody fragment; and

(b) reacting the aldehyde group of the resultant oxidized antibody or antibody fragment with a chelator-metal ion complex, said chelator-metal ion complex comprising a compatible chelator containing an amine group selected from primary amine, hydrazine, hydrazide, hydroxylamine, phenylhydrazine, semicarbazide and thiosemicarbazide groups coordinately bound to a metal ion, to form an antibody-chelator-metal ion complex having substantially the same immunoreactivity and immunospecificity as the unconjugated antibody or antibody fragment.

In this embodiment, the compatible chelator is coordinately bound to the metal ion prior to covalent attachment of the chelator to the antibody or antibody fragment.

The invention is also directed to intermediates and final products of the above-described methods. More specifically, this invention is directed to antibody-chelator conjugates comprising a compatible chelator attached through a covalent bond to a carbohydrate moiety of an oxidized antibody or antibody fragment, said antibody-chelator conjugate having substantially the same immunoreactivity and immunospecificity as the unconjugated antibody or antibody fragment. Additionally, the invention concerns antibody-metal ion complexes comprising an antibody-chelator conjugate comprising a compatible chelator attached through a covalent bond to a carbohydrate moiety of an oxidized antibody or antibody fragment, said antibody-chelator conjugate coordinately bound through said compatible chelator to a metal ion to form an antibody-chelator-metal ion complex having substantially the same immunoreactivity and immunospecificity as the unconjugated antibody or antibody fragment.

Independent of the method by which the antibody-chelator-metal ion complexes of the invention are made, it has been found that improved results may be obtained in ensuing uses of the complexes, particularly in vivo, when the complexes are purified to separate the antibody-chelator-metal ion complexes from non-chelated metal ions to obtain antibody-chelator-metal ion complexes substantially free of non-chelated metal ions. By "substantially free" of non-chelated metal ions is meant at least about 80-90 percent free of such ions. More specifically, the purified complexes of the invention contain metal ion chelated to the chelator, but are substantially free of metal ion adventitiously bound to antibody, that is, metal ion associated with the antibody protein or carbohydrate and not complexed to the chelator. The purification can be accomplished by any suitable purification method, including but not limited to high performance gel permeation liquid chromatography.

This purification step has the additional benefit of removing undesirable aggregates of antibodies prior to administration. Any such antibody aggregates or denatured antibodies would be taken up by the reticuloendothelial system for removal, and this transport away from the target site or specific tissue to be imaged would diminish the degree of localization or the quality of the image.

The antibody-chelator-metal ion complexes of the invention are ideally suited for high-resolution tissue imaging in vivo. Such imaging of specific tissue involves administering to a human an effective amount of an antibody-chelator-metal ion complex, wherein said antibody-chelator-metal ion complex is immunoreactive with and immunospecific for an antigenic determinant of said specific tissue and substantially non-immunoreactive with and non-immunospecific for non-specific tissue and said antigenic determinant is not found in substantial amount in non-specific tissue. As demonstrated in the examples hereafter, this method can be used to localize specific tissue with a minimum of "background" dispersal of metal ion.

Further in vivo uses of the antibody-chelator-metal ion complexes of the invention include therapeutic treatment of cellular disorders. Such methods comprise administering a therapeutically effective amount of an antibody-chelator-metal ion complex of the invention, said antibody-chelator-metal ion complex being immunoreactive with and immunospecific for a target site associated with said cellular disorder and substantially non-immunoreactive with and non-immunospecific for tissue not associated with said cellular disorder, and wherein the metal ion emits cytotoxic beta particles or alpha particles.

Finally, the complexes of the invention (hereinafter abbreviated as antibody-chelator-metal ion complexes) may also be used in methods for testing for antigen, which involve, for example, (a) mixing an antibody-chelator-metal ion complex with a sample suspected of containing a particular antigen, the antibody-chelator-metal ion complex being immunoreactive with and immunospecific for said antigen, and (b) detecting any interaction of said antibody-chelator-metal ion complex with any antigen in the sample.

## 5.1. ANTIBODIES

According to the present invention, antibodies directed against any antigen or hapten may be used. Although conventional antibodies (antisera) may be used, monoclonal antibodies offer several advantages.

Each monoclonal antibody is specific for one antigenic determinant. Additionally, large amounts of each monoclonal antibody can be produced.

Antibodies used in the present invention may be directed against any target, e.g., tumor, bacterial, fungal, viral, parasitic, mycoplasmal, histocompatability, differentiation and other cell membrane antigens, pathogen surface antigens, toxins, enzymes, allergens, drugs and any biologically active molecules.

Drugs of particular interest are opioids, amphetamines, barbiturates, steroids, catecholamines, dilantin, theophylline, histamine, cannabinoids, and the like. For a more complete list of antigens, see U.S. Patent 4,193,983, particularly columns 7-11, which patent specification is incorporated herein by reference. When it is desired to image cardiovascular disease or damage following, e.g., myocardial infarcts, antibodies directed against myosin or another cardiac cytosol protein may be used. Additionally, a combination of antibodies reactive to different antigenic determinants may be used. Immunoglobulins which may be used as carriers include: certain classes of antibodies such as IgA, IgD, IgE, IgM; certain classes of IgG; or certain fragments of immunoglobulins, e.g., half antibody molecules (a single heavy:light chain pair), or Fab, Fab', or $(Fab')_2$ fragments.

Use of antibody fragments may be advantageous for tissue imaging systems because these antibody fragments permeate target sites at an increased rate. The Fab' fragments of IgG immunoglobulins are obtained by cleaving the antibody molecule with pepsin (resulting in a bivalent fragment, $(Fab')_2$) or with papain (resulting in 2 univalent fragments, 2 Fab). Parham, 1983, J. Immunol. 131: 2895-2902; Lamoyi and Nisonoff, 1983, J. Immunol. Meth. 56: 235-243. The bivalent $(Fab')_2$ fragment can be split by mild reduction of one or a few disulfide bonds to yield univalent Fab' fragments. The Fab and $(Fab')_2$ fragments are smaller than a whole antibody molecule and, therefore, permeate the target site or tissue more easily This may offer an advantage for in vivo imaging since conjugates will more readily penetrate in vivo sites (e.g., tumor masses, infection sites, etc.). An additional advantage is obtained when using conjugates formed with antibody fragments because these fragments do not cross a placental barrier. As a result, using this embodiment of the invention, an in vivo site (such as a tumor) may be imaged in a pregnant female without exposing the fetus to the imaging compound.

5.2. COMPATIBLE CHELATORS

As used herein, the term "compatible chelator" means any compound that (a) is able to donate electrons and combine by coordinate bonding with a metal ion to form structures called chelates or chelation complexes and (b) is suitable for attachment to an antibody or antibody fragment without loss of ability to chelate metal ions or destruction of the immunoreactivity or immunospecificity of the antibody or antibody fragment. Where necessary, derivatives of known chelators may be synthesized such that the product is a compatible chelator suitable for attachment to antibodies or antibody fragments by methods of the invention.

Of additional interest are compatible chelators which have multiple sites for chelation of metal ions. For multiple site compatible chelators, a single covalent attachment to an antibody or antibody fragment would result in a conjugate capable of binding metal ion at a number of sites. Radioactive complexes of such conjugates would have high specific radioactivity, that is, high radioactivity per antibody molecule.

Alternatively, higher specific radioactivity (or higher ratios of metal ion to antibody molecule) can be achieved by attachment of a single site compatible chelator at a plurality of sites of the antibody or antibody fragment. This plurality of sites may be introduced into the antibody or antibody fragment by either of two methods. First, one may generate multiple aldehyde groups in the same antibody molecule. Second, one may attach to aldehyde of the antibody molecule a "branched linker" having multiple functional sites for subsequent attachment to compatible chelators. The functional sites of the branched linker may be aldehyde groups, or may be any chemical site to which compatible chelators may be attached. Still higher specific radioactivities may be obtained by combining these two approaches, that is, attaching multiple site compatible chelators at several sites on the antibody or antibody fragment.

Immediately below are identified some important classes of compatible chelators suitable for use in the present inventions. Compatible chelators include, but are not limited to, derivatives of diethylenetriaminepentaacetic acid, ethylenediaminetetraacetic acid (EDTA), dimercaptosuccinic acid, 2,3-dimercaptopropanesulfonic acid, metallothione in and cryptates, such as those recently described by Gansow, et al. (1981, J. Heterocyclic Chem. 18: 297).

5.2.1. COMPATIBLE CHELATORS FOR CARBOHYDRATE ATTACHMENT

As previously explained, compatible chelators are utilized to attach metal ions to antibody molecules.

According to the present invention, suitable compatible chelators for reaction with oxidized antibodies or oxidized antibody fragments include those containing an amine selected from the group consisting of primary amine, hydrazine, hydrazide, hydroxylamine, phenylhydrazine, semicarbazide and thiosemicarbazide groups.

Such reactive functional groups may exist as part of the structure of the chelator, or may be introduced by suitable chemistry on chelators not containing such groups.

For example, diethylenetriaminepentaacetic acid (DTPA) lacks the appropriate amine group for facile attachment to oxidized carbohydrate. However, chemical modification can produce a variety of suitable derivatives, such as amine-containing derivatives of mixed anhydrides of DTPA including, but not limited to p-aminoaniline-DTPA, hydrazide-DTPA, phenylhydrazide-DTPA, hydroxylamine-DTPA, semicarbazide-DTPA, thiosemicarbazide-DTPA, polyethyleneimine-DTPA, p-phenylenediamine-DTPA, DTPA mono[(4-aminophenyl)methyl] amide and amino acid-containing derivatives of DTPA, including, but not limited to α-N-DTPA-L-lysine, glycyl-tyrosyl-lysine-DTPA and L-lysine benzyl ester-DTPA.

## 5.3. METHODS OF ATTACHMENT TO ANTIBODIES AND ANTIBODY FRAGMENTS

According to the methods of the present invention, a compatible chelator (or compatible chelator-metal ion complex) is attached to an antibody directed against a target antigen, by attachment to the carbohydrate moieties of the antibody or antibody fragment

The attachment must not significantly change the essential characteristics of the antibody or antibody fragment, such as immunospecificity and immunoreactivity. Additional considerations include simplicity of reaction and stability of the antibody conjugate produced.

### 5.3.1. ATTACHMENT TO OXIDIZED CARBOHYDRATE MOIETIES

Glycoproteins are biologically important macromolecules which share structural characteristics including carbohydrate residues covalently attached to a polypeptide backbone. Since antibodies are glycoproteins, compounds may be attached to the carbohydrate moiety of the molecule. Some of the carbohydrate moieties are located on the Fc region of the immunoglobulin and are required in order for Cl binding to occur. The carbohydrate moiety of the Fc region of an immunoglobulin may be utilized in the scheme described herein. Alternatively, the Fab or Fab' fragments of any immunoglobulins which contain carbohydrate moieties may be utilized in the reaction scheme described herein. An example of such an immunoglobulin is the human IgM sequenced by Putnam, et al. (1973, Science 182: 287).

As explained in detail below, the carbohydrate side chains of antibodies or Fab or Fab' fragments may be selectively oxidized to generate aldehydes. The resulting aldehydes may then be reacted with amine groups (e.g., ammonia derivatives such as a primary amine, hydroxylamine, hydrazine, hydrazide, phenylhydrazine, semicarbazide or thiosemicarbazide) to form a Schiff base or reduced Schiff base (e.g., imine, enamine, oxime, hydrazone, phenylhydrazone, semicarbazone or thiosemicarbazone, or reduced forms thereof).

Alternatively, the carbohydrate moiety of the antibody may be modified by enzymatic techniques so as to enable attachment to or reaction with other chemical groups. One example of such an enzyme is galactose oxidase which oxidizes galactose in the presence of oxygen to form an aldehyde.

#### 5.3.1.1. CHEMICAL METHODS OF OXIDATION

Oxidation of the carbohydrate portion or moiety of antibody molecules leads to formation of aldehyde groups. A variety of oxidizing agents can be used, such as periodic acid, paraperiodic acid, sodium metaperiodate and potassium metaperiodate. Among these, oxygen acids and salts thereof are preferred since secondary or undesirable side reactions are less frequent. For a general discussion, see Jackson, 1944, Organic Reactions 2, p. 341; Bunton, 1965, Oxidation in Organic Chemistry, Vol. 1 (Wiberg, ed.), Academic Press, New York, p. 367.

Oxidation of antibodies with these oxidizing agents can be carried out by known methods. In the oxidation, the antibody is used generally in the form of an aqueous solution, the concentration being generally less than 100 mg/ml, preferably 1 to 20 mg/ml. When an oxygen acid or a salt thereof is used as the oxidizing agent, it is used generally in the form of an aqueous solution, and the concentration is generally 0.001 to 10mM and preferably 1.0 to 10mM. The amount of the oxygen acid or salt thereof depends on the kind of antibody, but generally it is used in excess, for example, twice to ten times as much as the amount of the oxidizable carbohydrate. The optimal amount, however, can be determined by routine

experimentation.

In the process for oxidizing antibodies with oxygen acids or salts thereof, the optional ranges include a pH of from about 4 to 8, a temperature of from 0 to 37° C, and a reaction period of from about 15 minutes to 12 hours.

During the oxidation of the glycoprotein with an oxygen acid or a salt thereof, light is preferably excluded to prevent over-oxidation of the glycoprotein.

5.3.1.2. ENZYMATIC METHODS OF OXIDATION

Oxidation of the carbohydrate portion of antibody molecules may also be done with the enzyme, galactose oxidase (Cooper, et al., 1959, J. Biol. Chem. 234: 445-448). The antibody is used in aqueous solution, the concentration being generally 0.5 to 20 mg/ml. The enzyme generally is used at about 5 to 100 units per ml of solution, at a pH ranging from about 5.5 to about 8.0. The influence of pH, substrate concentration, buffers and buffer concentrations on enzyme reaction are reported in Cooper, et al., supra.

5.3.1.3. PREPARATION OF ANTIBODY-CHELATOR CONJUGATES

The antibody-chelator conjugates (or antibody-metal ion complexes when pre-chelated metal ion is attached to the chelator prior to reaction of the chelator with antibody) of the invention may be produced by reacting the oxidized antibody with a compatible chelator having an available amine group selected from the group consisting of primary amine, hydrazine, hydrazide, hydroxylamine, phenylhydrazine, semicarbazide and thiosemicarbazide groups. The immediately resulting products (antibody-chelator conjugates or antibody-metal ion complexes) contain a carbon-nitrogen double bond resulting from elimination of a molecule of water from the initial addition products:

$$\text{Antibody-CH=O} + NH_2\text{-R} \rightarrow \text{Antibody-CH=N-R} + H_2O$$

For a general discussion of the reaction of aldehydes with hydrazides, see March, 1978, Advanced Organic Chemistry: Reactions, Mechanisms and Structure, McGraw-Hill Co., New York, p. 824-825.

A solution of the oxidized antibody at a concentration of from about 0.5 to 20 mg/ml is mixed with the compatible chelator (molar ratios of reactive chelator group to antibody aldehyde ranging from about 1 to about 10,000) and the solution incubated for from about 1 to 18 hours. Suitable temperatures are from 0 to 37° C and pH may be from about 6 to 8.

5.3.1.4. STABILIZATION OF THE ANTIBODY-CHELATOR CONJUGATES

After the antibody-chelator conjugates (or antibody-metal ion complexes) have been formed between the antibody and a compatible chelator as described in Section 5.3.1.3, they can optionally be stabilized with a suitable reducing agent, such as sodium cyanoborohydride or sodium borohydride:

$$\text{Antibody-CH=N-R} \xrightarrow[\text{agent}]{\text{reducing}} \text{Antibody-}CH_2\text{-NH-R}$$

Reducing agent is generally added to a molar excess of from about 10 to 100 fold molar excess over available aldehyde groups. For a general discussion, see Jentoft and Dearborn, 1979, J. Biol. Chem. 254: 4359.

5.4. PREPARATION OF COMPLEXES

Chelator-metal ion complexes may be prepared by attaching the metal ion directly to the chelator. Conventional methods of attaching metal ions to chelators may be utilized to accomplish binding to chelator. For example, $^{111}$Indium ($^{111}$In) may be attached to a water-soluble chelator compound by incubation of $^{111}In\text{-}Cl_3$ in sodium acetate with the chelator, e.g., for 1 hour at 37° C.

As explained previously, according to the present invention, the metal ion may be attached to the

chelator either before or after the chelator is attached to the antibody molecule. Whatever method of attachment is utilized, it may be significant for many applications that any metal ion not specifically attached to the chelator compound be removed before the complexes are used, particularly in vivo. According to the preferred embodiment, the non-specifically attached metal ion is removed using a high performance liquid molecular sieve chromatography system.

For in vitro detection systems, e.g., improved immunoradiometric assay systems, the antibody molecule or antibody fragment may be attached to a compatible chelator according to methods described herein, and the antibody-chelator conjugate intermediate may be purified, if necessary, and stored frozen at -20 °C until needed. The metal ion may then be attached to the antibody-chelator conjugate. For instance, $^{111}$Indium may be attached by incubation of $^{111}InCl_3$ with the antibody-chelator conjugate in acetate buffer, pH 6.0, at 37 °C for 1 hour. Such method of attachment advantageously allows purification of the antibody-chelator intermediate without exposing the preparer to excessive handling of radioactive materials, and without necessity of lengthy storage of radioisotopes in the work areas.

## 5.5. USES OF ANTIBODY-METAL ION COMPLEXES

The antibody-metal ion complexes of the invention are useful in a variety of therapeutic and in vitro and in vivo diagnostic applications.

Throughout this application the term "cellular disorder" is meant to include all neoplasms, including cancers, adenomas, and hyperplasias; certain immunological disorders, including autoimmune diseases, graft-versus-host diseases (e.g., after bone marrow transplantation), immune suppressive diseases, e.g., after kidney or bone marrow transplantation. Treatment of such cellular disorders involving, for example, bone marrow transplantation, may include purging (by killing) undesired cells, e.g., malignant cells or mature T lymphocytes.

### 5.5.1. IN VIVO THERAPEUTICS

Therapeutic applications center generally on treatment of various cellular disorders, including those broadly described above, by administering an effective amount of the antibody-metal ion complexes of the invention. The properties of the antibody in being immunospecific for and immunoreactive with a particular antigen render it ideally suited for delivering metal ions to specific cells, tissues, organs or any other site having that particular antigen.

According to this aspect of the invention, the antibody or antibody fragment of the antibody-metal ion complex functions to deliver the complex to the target site, at which site the metal ion, which is chosen for its cell killing properties, is able to destroy nearby cells. Suitable beta emitting ions for therapeutic uses include: $^{46}$Scandium, $^{47}$Scandium, $^{48}$Scandium, $^{72}$Gallium and $^{73}$Gallium. Suitable alpha emitting metal ions include those with a relatively short half-life of about four days or less, including $^{211}$Bismuth, $^{212}$Bismuth, $^{213}$Bismuth and $^{214}$Bismuth, with $^{212}$Bismuth being preferred.

In vivo administration may involve use of pharmaceutical compositions of antibody-metal ion complex in any suitable carrier, including serum or physiological saline, with or without another protein, such as human serum albumin. Dosage of the complexes may readily be determined by one of ordinary skill, and may differ depending upon the nature of the cellular disorder and the metal ion used. Route of administration may be parenteral, with intravenous administration generally preferred.

### 5.5.2. IN VIVO DIAGNOSTICS

In vivo diagnostic applications involve imaging of specific tissues or cellular disorders by administration of a sufficient amount of the antibody-metal ion complexes of the invention to enable the complexes to localize at the tissue in the appropriate time frame. Dosage and other aspects of administration of the complexes in vivo are generally discussed in the preceding section.

A wide variety of metal ions suitable for in vivo tissue imaging have been tested and utilized clinically. For imaging with radioisotopes the following characteristics are generally recognized as desirable and/or necessary: (a) low radiation dose to the patient; (b) high photon yield which permits a nuclear medicine procedure to be performed in a short time period; (c) ability to be produced in sufficient quanitites; (d) acceptable cost; (e) simple preparation for administration; and (f) no requirement for subsequent isolation of the patient. These characteristics generally translate into the following: (a) the radiation exposure to the most critical organ is less than 5 rad; (b) a single image can be otained in several hours; (c) the radioisotope does not decay by emission of a particle (e.g., $\beta^-$ or $B^+$); (d) the isotope can be readily

detected; and (e) the half-life is less than four days. (Lamb and Kramer, "Commercial Production of Radioisotopes for Nuclear Medicine", IN Radiotracers For Medical Applications, Vol. 1, Rayudu (ed.), CRC Press, Inc., Boca Raton, pp. 17-62.)

An alternative method for imaging with radioisotopes involves positron emission tomography. Suitable positron emitting isotopes include $^{43}$Scandium, $^{44}$Scandium, $^{52}$Iron, $^{55}$Cobalt and $^{68}$Gallium.

Tissue imaging may also utilize nonradioactive paramagnetic metal ions such as $^{54}$Iron, $^{56}$Iron, $^{57}$Iron, $^{58}$Iron, $^{157}$Gadolinium and $^{55}$Manganese, which are detectible by nuclear magnetic resonance spectroscopy.

Tissues which one may image include any solid neoplasm, certain organs such as lymph nodes, parathyroids, spleen and kidney, sites of inflammation or infection (e.g., macrophages at such sites), myocardial infarction or thromboses (neoantigenic determinants on fibrin or platelets), etc.

### 5.5.3. IN VITRO DIAGNOSTICS

In vitro analytical procedures for detecting a particular antigen using the antibody-metal ion complexes of the invention employ standard immunoradiometric assay techniques. For a general review of such techniques, see Hales and Woodhead, Methods in Enzymology 70: 334-355 (1980). Generally, immunoradiometric assays (IRA) involve labeled antibodies to detect unlabeled antigens. Numerous variations of the immunoradiometric assay have been developed, including for example, the two-site IRA and the indirect IRA. These two methods are discussed generally below.

The objective of the two-site IRA is to use a specific antibody on a solid phase, e.g., cellulose or Sepharose, to extract antigen. While the antibody remains bound to the solid phase, a second labeled antibody is bound to another site on the antigen. The second antigen can then be measured as a function of the amount of bound labeled antibody. In this method the antigen is bound to two different antibodies at two sites on the antigen. In another method purified antigen is adsorbed or coupled to a solid phase support.

In the indirect IRA the (first) antibody used for measuring antigen is indirectly labeled through the use of a labeled antibody to the immunoglobulin of the same species as that in which the first antibody is raised. This labeled anti-immunoglobulin antibody is then reacted with the first antibody and antigen. The labeled antibody can also be used in the conventional or two site IRAs.

These diagnostic techniques and others are intended to be encompassed by the present invention.

In the context of this invention, all of these IRA methods for testing for antigen have in common the following two steps: (a) mixing an antibody-metal ion complex with a sample suspected of containing the antigen, and (b) detecting the interaction, if any antigen is present, of said complex with the antigen.

For in vitro diagnostics, all of the gamma and positron emitting metal ions, as well as the paramagnetic metal ions in Section 5.5.2, as well as $^{153}$Gadolinium are suitable. For fluorescence diagnostic assays, lanthanides may be employed, including Praseodymium, Neodymium, Samarium, Europium, Gadolinium, Terbium, Dysprosium, Holmium, Erbium, Thulium and Ytterbium.

## 6. EXAMPLES: PREPARATION OF ANTIBODY-METAL ION COMPLEXES VIA CARBOHYDRATE ATTACHMENT

The following experiments demonstrate the formation of specifically radiolabeled antibody-metal ion complexes which according to the instant inventions are useful for in vivo imaging systems, for in vivo therapy and for in vitro detection systems.

### 6.1. PREPARATION OF COMPATIBLE CHELATORS

As described above, compatible chelators are utilized to attach a metal ion, such as a radioisotope, to an antibody or antibody fragment. The following examples illustrate preparation of suitable compatible chelators.

In all the following examples the water used as a solvent or to form solvent systems and buffers was first passed through a Chelex® resin-containing column (BioRad Labratories, Inc., Richmond, CA) and stored in acid-washed containers.

#### 6.1.1. DIETHYLENETRIAMINEPENTAACETIC ACID ANHYDRIDE

The mixed anhydride of diethylenetriaminepentaacetic acid (DTPA mixed anhydride) was prepared according to the method described by Krejcarek and Tucker, 1977, Biochem. Biophys. Res. Commun. 77: 581-585. Briefly, 2.54 mmole DTPA and 12.7 mmole triethylamine were dissolved in 20 ml of water and the

solution dried under vacuum to yield the DTPA salt. The salt was then dissolved in 20 ml of a solvent system consisting of acetonitrile-dimethylformamide ($CH_3CN$-DMF) in a ratio of 65:35, and cooled to -5°C in an ice-rock salt bath. After the addition of 2.54 mmole isobutylchloroformate, the solution was stirred under nitrogen for twenty minutes. The DTPA mixed anhydride product which remained in solution could be separated from the precipitated triethylamine hydrochloride either by filtering or by centrifuging and decanting the supernatant.

6.1.2. P-AMINOANILINE-DIETHYLENETRIAMINEPENTAACETIC ACID

P-Aminoaniline-diethylenetriaminepentaacetic acid anhydride (ADTPA) was prepared as follows. Briefly, 25.4 mmole p-phenylenediamine in 50 ml of a solvent system containing $CH_3CN$-DMF (65:35) wag added to 2.0 g of fresh DTPA mixed anhydride prepared as described in Section 6.1.1. The solution was maintained with stirring at -5°C for 2 hours under nitrogen, and then at room temperature (approximately 24°C) overnight. At the end of this time period, the initially homogeneous solution had become a heterogeneous mixture. This heterogeneous reaction mixture was then evaporated to dryness under vacuum, and the resulting residue dissolved in a small aliquot (50 ml) of distilled water. The pH of the reaction mixture was adjusted to 11.0 using sodium hydroxide (NaOH) solution (6M). Unreacted p-phenylenediamine was removed by extraction with methylene chloride. The aqueous phase was collected, concentrated under vacuum and lyophilized to yield a pale (white) powder of crude ADTPA. The crude ADTPA was purified by elution through a packed silica gel column using ethanol-aqueous ammonia (4:1), followed by distilled water.

6.1.3. α-N-DIETHYLENETRIAMINEPENTAACETIC ACID L-LYSINE

α-N-Diethylenetriaminepentaacetic acid L-lysine (KDTPA) was prepared as follows: 2.26 mmole of N-ϵ carbobenzoxy-L-lysine benzyl ester (Vega Biochemicals, Tucson, AZ) was reacted with 2.71 mmole triethylamine in 40 ml of acetonitrile, while maintained with stirring at room temperature for 30 minutes under nitrogen. Then 2.26 mmole of freshly prepared DTPA mixed anhydride in 20 ml $CH_3CN$-DMF (65:35) was added. The reaction mixture was maintained with stirring under nitrogen for 2 hours at 0°C, and then overnight at room temperature. The solution was evaporated to dryness under vacuum and the resulting residue dissolved in 50 ml distilled water. The unreacted lysine ester was removed by adjusting the pH to 11.0 with NaOH solution and extracting with methylene chloride. The aqueous solution was then concentrated under vacuum and the pH adjusted to neutrality with a small aliquot of hydrochloric acid (HCl). The carbobenzoxy protecting group was then removed using palladium/activated charcoal (5% palladium) under 2.07 bar (30 lb-inch$^2$) hydrogen for 4 hours in a Parr Hydrogenator. The resulting mixture was filtered through Celite®, and the filtrate lyophilized to yield a white powder of crude KDTPA. This crude product was further purified to yield KDTPA by elution through a silica gel column with ethanol-aqueous ammonia (4:1), followed by distilled water.

6.1.4. HYDRAZIDE-DIETHYLENETRIAMINEPENTAACETIC ACID

The hydrazide derivative of DTPA (HDTPA) was prepared as follows: 6.3 mmole of anhydrous hydrazine was dissolved in 2 ml dimethylformamide (DMF) and reacted with 2.54 mmole DTPA mixed anhydride in 20 ml $CH_3CN$-DMF (65:35). The heterogeneous mixture was maintained with stirring at 4°C for 1 hour, and then at room temperature for 1 hour. The reaction mixture was evaporated to dryness under vacuum, and the residue dissolved in ethanol-aqueous ammonia (4:1). The product was then purified by elution through silica gel with ethanol-aqueous ammonia, followed by distilled water. Product-containing fractions were collected, and the pH adjusted to 3.0 using a small aliquot of hydrochloric acid. The solution was filtered and the filtrate lyophilized to yield HDTPA.

6.1.5. GLYCYL-TYROSYL-LYSINE-DIETHYLENETRIAMINEPENTAACETIC ACID

The glycyl-tyrosyl-lysine-diethylenetriaminepentaacetic acid anhydride (GYK-DTPA) was prepared as follows. The initial peptide reactant N-FMOC-glycyl-(o-benzyl-tyrosyl-(ϵ-N-carbobenzoxy)) lysine was prepared according to standard solid phase synthetic methods described by Baranz and Merrifield, IN The Peptides, Vol. 2, Gross and Meienhoffer (ed.), Acad. Press, New York, pp. 3-385. The derivatized peptide was cleaved from the resin and partially deblocked by bubbling hydrogen bromide (HBr) gas through a suspension of the reaction mixture in trifluoroacetic acid containing methoxybenzene (a fifty-fold molar excess over tyrosine) for 60 minutes at room temperature. The resin was removed by filtration and the

filtrate evaporated to dryness. The residue was dissolved in a minimum amount of methanol, and the product precipitated with ether and used without further purification.

One mole N-FMOC-glycyl-tyrosyl-lysine in DMF was reacted with 1 mole DTPA mixed anhydride, prepared as described in Section 6.1.1 except that diisopropylethylamine was used in place of triethylamine, for 30 minutes at -15°C, and then maintained at room temperature for 1 hour. The solvent was removed by rotary evaporation, and the oily residue dissolved in a small aliquot of DMF. Distilled water was added to precipitate the FMOC-GYK-DTPA produced. The FMOC group was removed by addition of an equal volume of 40% dimethylamine to a solution of FMOC-GYK-DTPA in DMF, followed by incubation for 30 minutes at room temperature. The solvent was evaporated to dryness and the residue taken up in distilled water. The crude product was purified by extraction with ethyl acetate, and the resulting aqueous solution of GYK-DTPA was lyophilized to dryness.

6.1.6. POLYETHYLENEIMINE-DIETHYLENETRIAMINEPENTAACETIC ACID

Polyethyleneimine-diethylenetriaminepentaacetic acid (PEI-DTPA) was prepared as follows: 2.54 mmole DTPA was dissolved in 100 ml $H_2O$, and the pH was adjusted to 5.0 using 6M NaOH solution. Then 12.7 mmole 1-ethyl-3-(3-dimethylaminopropyl) carbodiimide (Sigma Chemical Co., St. Louis, MO) and 144 $\mu$mole polyethyleneimine (Polysciences, Inc. Warrington, PA) were added. The pH was readjusted to 5.0 using aqueous HCl, and the solution was maintained with stirring at room temperature overnight. The PEI-DTPA formed was separated from the reaction mixture either by reverse phase chromatography or by gel filtration.

6.1.7. DIETHYLENETRIAMINEPENTAACETIC ACID MONO[(4-AMINOPHENYL)METHYL]AMIDE

P-Nitrobenzylamine HCl (174 mg, 0.92 mmole) was treated with 3 ml of 0.3N NaOH to form yellow oil and the oil was extracted with $CH_2Cl_2$. The organic layer was separated and dried in vacuo to obtain the p-nitrobenzylamine free base.

DTPA anhydride (CDTPA) was synthesized according to the procedure of Hnatowich, et al. (1982, Int. J. Appl. Radiat. Isot. 33: 327-332).

A solution of CDTPA (1.64 g, 4.6 mmole in 150 ml of dry dimethylformamide (DMF)) was added to the p-nitrobenzylamine and warmed to 60°C under $N_2$ gas. After stirring for 24 hours, thin layer chromatography showed no unreacted p-nitrobenzylamine in the solution. The solution was dried in vacuo and hydrolyzed with saturated $NaHCO_3$ solution. The p-nitrobenzylamine-DTPA adduct was separated from the reaction mixture using $C_{18}$ reversed phase high performance liquid chromatography (HPLC). It was then reduced using a Parr Hydrogenator in the presence of 5% palladium/activated charcoal under 2.07 bar (30 lbs/in$^2$)$H_2$ gas for 4 hours. The solution was dried by rotary evaporation and the oil was triturated with acetone-ether (4:1) to yield a pale yellow product.

6.1.8. L-LYSINE BENZYL ESTER-DIETHYLENETRIAMINEPENTAACETIC ACID

N-$\epsilon$-Z-L-Pentaacetic Acid lysine benzyl ester HCl (0.5 g, 1.12 mmole, Vega Chemical Co., Tucson, AZ) was adjusted to pH 9.0 with 5% $NaHCO_3$ and 0.1N NaOH solutions. The aqueous solution was extracted with $CH_2Cl_2$ and the organic layer was separated. The organic solution was dried over sodium sulfate and then was filtered, the filtrate evaporated to dryness in vacuo, obtaining the free base form of N-$\epsilon$-Z-L-lysine benzyl ester.

DTPA anhydride (CDTPA) was synthesized according to the procedure of Hnatowich, et al. (1982, Int. J. Appl. Radiat. Isot. 33: 327-332).

To a solution of 2.19 g (6.15 mmole) of CDTPA in 200 ml of dry dimethylformamide was added the above prepared N-$\epsilon$-Z-L-lysine benzyl ester free base and warmed to 60°C under $N_2$ gas for 24 hours. All lysine ester was reacted with CDTPA from thin layer chromatography analysis. The reaction mixture was dried in vacuo and the unreacted anhydride was hydrolyzed with saturated $NaHCO_3$ solution. The N-$\epsilon$-Z-L-lysine benzyl ester-DTPA adduct was isolated from the mixture using C18 reversed phase HPLC. The CBZ protecting group was removed by hydrogenolysis in a Parr Hydrogenator in the presence of 5% palladium/activated charcoal and 2.07 bar (30 lbs/in$^2$)$H_2$ gas for 5 hours. The solution was dried and the oily residue was triturated with acetone-ether (4:1) solvent mixture to yield the solid product.

6.2. ATTACHMENT OF CHELATOR TO ANTIBODY AND METAL ION

The specific monoclonal antibody utilized in the following experiments for injection into experimental

animals was a rat IgG antibody specific for a Class I major histocompatability complex (MHC) antigen of Brown Norway (BN) rats. This antibody, designated CYT-015, was prepared by fusion of spleen cells from Lewis rats previously immunized by injection with cells from BN rats, with myeloma cell line SP2/0 AG14 according to the method described by McKearn, et al. (1979, Immunol. Rev. 47: 91-115). After cloning, CYT-015-containing ascites were generated by intraperitoneal injection of cells into pristane-primed nude mice. IgG monoclonal antibody was purified from this ascites by specific elution on a Protein-A-Sepharose column (Pharmacia Fine Chemicals, Piscataway, NJ).

A mouse monoclonal IgG antibody specific for a monomorphic determinant of a human Class I major histocompatibility complex was used as a carrier antibody for conjugates injected into control animals. This antibody is designated CYT-012.

### 6.2.1. FORMATION OF ANTIBODY-CHELATOR CONJUGATES

Radiolabeled antibody-metal ion complexes were prepared according to one method of the instant invention by first forming antibody-chelator conjugates. The carbohydrate moiety of the antibody (CYT-012 or CYT-015) was oxidized by reacting approximately 1 mg/ml glycoprotein in phosphate buffered saline (PBS) with 10 mM sodium metaperiodate ($NaIO_4$) (pH 6.0) for 1 hour on ice in the dark. The sample was then passed through a Sephadex® G-25 column (Pharmacia Fine Chemicals, Piscataway, NJ) and the protein fractions pooled. The oxidized antibody was then attached to a compatible chelator and either used immediately or stored frozen at -20°C.

For example, glycyl-tyrosyl-lysine-diethylenetriaminepentaacetic acid (GYK-DTPA) was coupled to oxidized antibody by incubating an aliquot of the antibody with a 200-fold molar excess of GYK-DTPA in PBS (pH 6.0) for 30 minutes at room temperature. To stabilize the product sodium cyanoborohydride ($NaCNBH_3$) was added to a final concentration of 10mM, and the reaction mixture was maintained at room temperature for a period ranging from 2 hours to overnight. The sample was then passed through a Sephadex® G-50 column (Pharmacia Fine Chemicals, Piscataway, NJ) and the protein fractions were pooled.

In other experiments, oxidized antibody was coupled either to p-aminoaniline-diethylenetriaminepentaacetic acid (ADTPA) or polyethyleneimine-diethyenetriaminepentaacetic acid (PEI-DTPA) by the same method except that a 2000-fold molar excess of ADTPA or PEI-DTPA was utilized.

### 6.2.2. FORMATION OF ANTIBODY-METAL ION COMPLEXES

Radioactive metal ion was then attached to the antibody-chelator conjugates in order to form radioactive antibody-metal ion complexes useful for imaging systems.

For example, 20 µl of stock $^{111}$Indium chloride ($^{111}In\text{-}Cl_3$) (New England Nuclear, Boston, MA) representing 1-2 mCi (3.7-7.4x$10^7$ Bq) was added to 20 µl 0.5M sodium acetate (pH 6.0), and incubated with antibody-chelator conjugate prepared as described above (50-200 µg) for 1 hour at 37°C. The sample was then eluted through a Sephadex® G-50 column (Pharmacia Fine Chemicals, Piscataway, NJ) and the protein fractions pooled. The pooled $^{111}$In-labeled antibody-metal ion complex was rechromatographed through a Waters Protein Pak 300SW high performance liquid chromatography (HPLC) column (Waters Associates, Milford, MA), and fractions were collected. The fraction corresponding to the elution volume of IgG in molecular weight represents the antibody-metal ion complex.

FIG. 1, for example, illustrates an HPLC chromatogram obtained during purification and isolation of $^{111}$Indium-labeled anti-Brown Norway major histocompatibility complex antibody-metal ion complex, $^{111}$In-CYT-015-ADTPA. As shown in FIG. 1, the optical density of the fraction containing peak radioactivity reveals the similarity of the antibody-metal ion complex to monomeric unconjugated IgG.

## 7. EXAMPLES: PREPARATION OF ANTIBODY-METAL ION COMPLEXES VIA CARBOHYDRATE ATTACHMENTS

The following examples illustrate the formation of compatible chelators and radiolabeled antibody-metal ion complexes which according to the instant invention are useful for in vivo imaging systems, for in vivo therapy and for in vitro detection systems.

### 7.1. HYDRAZIDE-FICOLL

Ficoll 70 was obtained from Pharmacia Fine Chemicals, Inc. (Piscataway, New Jersey). The carboxymethyl derivative of Ficoll 70 (CM-Ficoll) was prepared exactly as described by Inman (1975, J. Immunol.

114: 704). Two grams of CM-Ficoll were dissolved in 100 ml of water and 10 g of adipic dihydrazide were added slowly. The pH was adjusted to 4.7 by the dropwise addition of 1N HCl and then 1.25 g of 1-ethyl-3-(3-dimethyl-aminopropyl)carbodiimide were added and the pH was adjusted to 4.7 with 1N HCl. The reaction mixture was then stirred for 20 hours at 23-25 °C. The crude reaction product was purified by gel filtration chromatography on a 4.5 x 55 cm column of Sephadex® G-25. The column was eluted with phosphate buffered saline (PBS, 0.01 M sodium phosphate, 0.15 M sodium chloride, pH 7.4) and the void volume fractions were saved. The hydrazide-Ficoll was then dialyzed against water and lyophilized. The final product contained about 150 moles of hydrazide/mole of Ficoll.

7.2. (4-IODOACETYL)-AMINOBENZOICHYDRAZIDE-FICOLL

Thirty milligrams of N-succinimidyl-(4-iodoacetyl)aminobenzoate (SIAB, Pierce Chemical Co., Rockford, IL) were dissolved in 3 ml of acetonitrile. Six 0.48 ml aliquots of the SIAB solution were added to 20 ml of a 5 mg/ml solution of hydrazide-Ficoll (prepared as described in Section 7.1) dissolved in 0.1 M sodium phosphate buffer, pH 7.0, at 30 minute intervals. Before the second addition of SIAB solution, 5 ml of tetrahydrofuran were added to the hydrazide-Ficoll to clarify the solution. The reaction mixture was then stirred for 20 hours at 23-25 °C. The organic solvents were removed by bubbling $N_2$ gas through the reaction mixture and the resultant cloudy solution was clarified by centrifugation. The clear supernatant was lyophilized. Excess SIAB was removed by extracting the dry powder with tetrahydrofuran and the excess solvent was removed by evaporation under reduced pressure. The dry powder was dissolved in 5 ml of water and dialyzed for 4 hours at 4 °C. The (4-iodoacetyl)-aminobenzoichydrazide-Ficoll was stored frozen at -70 °C and contained about 16 iodoacetyl groups per mole of hydrazide-Ficoll.

7.3. 2-PYRIDYL DISULFIDE HYDRAZIDE DTPA

Ten milligrams of hydrazide-DTPA (prepared as described in Section 6.1.4) was dissolved in 0.1 ml of 0.2 M sodium bicarbonate buffer, pH 9.5. One-tenth milliliter of 0.1 M sodium phosphate buffer, pH 7.2, was added and the pH of the resultant solution was adjusted to 8.0; 0.3 ml of tetrahydrofuran and 0.1 ml of N,N-dimethylformamide were then added. One hundred milligrams of N-succinimdyl-3-(2-pyridyl-dithio)-propionate (Pharmacia Fine Chemicals, Inc., Piscataway, NJ) dissolved in 1.0 ml of tetrahydrofuran were added to the hydrazide-DTPA solution and the reaction mixture was stirred for 16 hours at 23-25 °C. The tetrahydrofuran was removed by bubbling $N_2$ through the solution and the resultant suspension clarified by centrifugation. Unwanted reaction products were precipitated by the addition of 1 ml of water, and the clear supernatant was further purified by repeated gel filtration on columns of Bio-Gel P-2 (BioRad Laboratories, Richmond, CA) and eluted with water. The product contained about 0.15-0.20 moles of 2-pyridyl-disulfide/mole of DTPA.

7.4. 3-MERCAPTOPROPIONICHYDRAZIDE-DTPA-$^{153}$Gd

One milligram of 2-pyridyl disulfide hydrazide DTPA (PDS-DTPA, prepared as described in Section 7.3), was dissolved in 0.1 ml of water and labelled with $8.9 \cdot 10^5$ Bq (0.024 mCi) of $^{153}GdCl_3$ (Amersham Corporation, Arlington Heights, IL) dissolved in 0.1 ml of 0.05 M HCl. The PDS-DTPA-$^{153}$Gd was then reduced with 5mM dithiothreitol and the resultant 3-mercoptopropionichydrazide-DTPA-$^{153}$Gd was purified by gel filtration on Sephadex® G-10.

7.5. $^{153}$Gd-DTPA-HYDRAZIDE-THIOACETYL-AMINOBENZOICHYDRAZIDE-FICOLL-ACETYLTHIO-IgG

One milligram of mouse anti-N. gonorrhoeae monoclonal IgG was diluted into 0.25 ml of PBS, pH 7.4. The IgG solution was then reduced with 5 mM dithiothreitol for 30 minutes at 23-25 °C. The reduced antibody was then passed over a 1x19 cm Sephadex® G-50 column and eluted with 0.1 M tris-(hydroxymethyl)-aminomethane buffer, pH 8.0, containing 1mM ethylenediaminetetracetic acid (Tris/EDTA). One milligram of (4-iodoacetyl)-aminobenzoichydrazide-Ficoll (prepared as described in Section 7.2) was added to the reduced antibody and the reaction mixture was incubated at 4C for 16 hours to form an iodoacetyl-aminobenzoichydrazide-Ficoll-acetylthio-IgG conjugate.

0.20 milligrams of 3-mercaptopropionichydrazide-DTPA-$^{153}$Gd (prepared as described in Section 7.4) were added to the iodoacetyl-aminobenzoichydrazide-Ficoll-acetylthio-IgG conjugate and the reaction mixture incubated at 4 °C for 16 hours. The $^{153}$GD-DTPA-hydrazide-thioacetyl-aminobenzoichydrazide-Ficoll-acetylthio-IgG complex was purified by immuno-adsorbtion with a resin composed of sheep anti-mouse IgG

antibody (Cooper Biomedical, Scientific Division, Malvern, PA) covalently coupled to cyanogen bromide activated Sepharose 4B (Sigma Chemical Co., St. Louis, MO). The bound complex was eluted with 0.1 M glycine buffer, pH 2.0 and desalted into Tris/EDTA buffer using Sephadex® G-25.

This is an example of the preparation of an antibody-metal ion complex formed by attachment of a single site compatible chelator at a plurality of sites introduced at the sulfhydryl groups of antibodies, as described in Section 5.2.

### 7.6. (4-IODOACETYL)-AMINOBENZOICHYDRAZIDE-FICOLL-IgG

One-half milligram of mouse anti-N. gonorrhoeae monoclonal IgG was oxidized by the method of Section 6.2.1. After dialysis versus PBS, pH 7.4, the antibody was diluted with PBS, pH 7.4 to a final volume of 0.577 ml. 0.702 ml of (4-iodoacetyl)-aminobenzoichydrazide-Ficoll solution (16.7 mg/ml, prepared as described in Section 7.2), were added along with 0.128 ml of 110 mM sodium cyanoborohydride solution dissolved in 0.1 M sodium phosphate, pH 6.0. The reaction mixture was allowed to incubate for 2 hours at 23-25 °C. The (4-iodoacetyl)-aminobenzoichydrazide-Ficoll-IgG conjugate was purified by immunoadsorption with a resin composed of sheep anti-mouse IgG (Cooper Biomedical, Scientific Division, Malvern, PA) covalently coupled to cyanogen bromide activated Sepharose 4B (Sigma Chemical Co., St. Louis, MO). The bound conjugate was eluted with 0.1 M glycine buffer, pH 2.0 and desalted into 0.1 M Tris(hydroxymethyl)-aminomethane, pH 8.0, containing 1 mM ethylenediaminetetraacetic acid using Sephadex® G-25. The purified conjugate contained about 2-3 moles of (4-iodoacetyl)-aminobenzoichydrazide-Ficoll/mole of IgG and was stored at -70 °C.

### 7.7. $^{153}$Gd-DTPA-HYDRAZIDE THIOACETYL AMINOBENZOICHYDRAZIDE-FICOLL-IgG

0.081 milligrams of 3-mercaptopropionic-hydrazide-DTPA-$^{153}$Gd (prepared as described in Section 7.4) were added to 0.050 mg of (4-iodoacetyl)-amino-benzoichydrazide-Ficoll-IgG prepared as described in Section 7.6. and the reaction mixture was incubated overnight at 4 °C. The free 3-mercaptoproprionichydrazide-DTPA-$^{153}$Gd was removed by chromatography on Sephadex® G-50. The resultant $^{153}$Gd-DTPA-hydrazide thioacetyl aminobenzoichydrazide Ficoll-IgG complex contained 13.5 moles of DTPA/mole of IgG.

This is an example of the preparation of an antibody-metal ion complex formed by attachment of a single site compatible chelator at a plurality of sites introduced at the carbohydrate moieties of antibodies as described in Section 5.2.

### 7.8. MALEIMIDE HYDRAZIDE FICOLL

Twenty-two milligrams of 4-(N-maleimidomethyl)-cyclohexane-1-carboxylic acid N-hydroxysuccinimide ester (SMCC, Sigma Chemical Co., St. Louis, MO) were dissolved in 3 ml of tetrahydrofuran. Six 0.5 ml aliquots of the SMCC solution were added to 20 ml of a 5 mg/ml solution of hydrazide-Ficoll (prepared as described in Section 7.1), dissolved in 0.1 M sodium phosphate buffer, pH 6.8, at 30 minute intervals. Two milliliters of tetrahydrofuran were added to the reaction mixture before the second and third additions of SMCC solution. The organic solvents were then removed by bubbling $N_2$ through the reaction mixture and the resultant cloudy solution was clarified by centrifugation. The clear supernatant was lyophilized and the excess SMCC was extracted with tetrahydrofuran. The organic solvent was then removed by evaporation under reduced pressure. The final product contained about 15 moles of maleimide/mole of Ficoll. This compound can be used in the same way as the (4-iodoacetyl)-aminobenzoichydrazide-Ficoll prepared by the method of Section 7.2.

## 8. EXAMPLES: IMAGING USING RADIOLABELED ANTIBODY-METAL ION COMPLEXES

The following examples illustrate methods for in vivo imaging to locate specific tissue or organ components utilizing the radiolabeled antibody-metal ion complexes prepared according to the instant invention.

### 8.1. TUMOR IMAGING

In one series of experiments, radiolabeled antibody-metal ion complexes were utilized to specifically locate tumorous tissues in experimental animals using a radioimaging system.

Nude mice were injected subcutaneously in the left hindquarter with 1 X $10^6$ BN rat lymphoma cells. Seven days post-injection when tumor sizes ranged from 2-4 mm in diameter, the animals received a retro-orbital sinus or intravenous injection of 10 $\mu g$ $^{111}$Indium-labeled anti-BN major histocompatability complex (MHC) antibody-metal ion complex, $^{111}$In-CYT-015-ADTPA. No animal received more than a single injection with complex.

Two groups of animals served as controls. One control group of non-tumor-bearing nude mice were injected either in the retro-orbital sinus or intravenously with 10 $\mu g$ $^{111}$In-CYT-015-ADTPA. Another control group of nude mice bearing BN tumors were injected with 10 $\mu g$ $^{111}$In-CYT-012-ADTPA.

Radioimaging was accomplished as follows: at 24, 48 or 72 hours post-injection with radiolabeled antibody-metal ion complex, animals were placed directly upon the inverted face of a gamma camera (General Electric Maxi Camera 37 interfaced with an ADEC Clinical Data System). Ten thousand - 25,000 counts were routinely accumulated on exposed X-ray film without correction for background radiation.

FIG. 2 illustrates in vivo images of radioactivity accumulated within BN tumor-bearing nude mice injected with $^{111}$In-CYT-015-ADTPA. Images A, B, C and D were taken 24, 24, 48 and 72 hours after injection, respectively. FIG. 2 clearly demonstrates that the complexes of the invention are specifically localized at the intended target with extremely low levels of non-specific localization.

FIG. 3 illustrates in vivo images of radioactivity in BN tumor-bearing control mice described above. Images E and F were taken 24 and 72 hours after injection, respectively. As shown, in FIG. 3 there was no detectable localization of radioactivity in BN tumor-bearing nude mice injected with $^{111}$In-CYT-012-ADTPA in which the antibody is specific for Class I human MHC antigen.

FIG. 4. presents in vivo images of non-tumor-bearing nude mice injected with $^{111}$In-CYT-012-ADTPA. Images G and H were taken 24 and 72 hours after injection, respectively. As shown in FIG. 4, there was also no detectable localization of radioactivity in non-tumor-bearing nude mice injected with radiolabeled antibody-metal ion complex, $^{111}$In-CYT-015-ADTPA, specific for BN tumor antigen.

### 8.2. RENAL TRANSPLANT IMAGING

In another series of experiments, radiolabeled antibody-metal ion complexes were utilized to specifically locate transplanted renal tissues by in vivo radioimaging.

Experimental rats bearing a transplanted functioning kidney or renal allograft were prepared as follows: the left kidney of a Lewis rat was surgically removed and a kidney from a BN x Lewis F1 rat was implanted in its place. The recipients' immune response against the foreign kidney transplant was chemically suppressed (see Stuart, et al., 1980, Immunol. Rev. 49: 127-165). Thus, the recipient rats possessed two functioning kidneys, only one of which carried the BN MHC antigen specifically recognized by CYT-015 antibody.

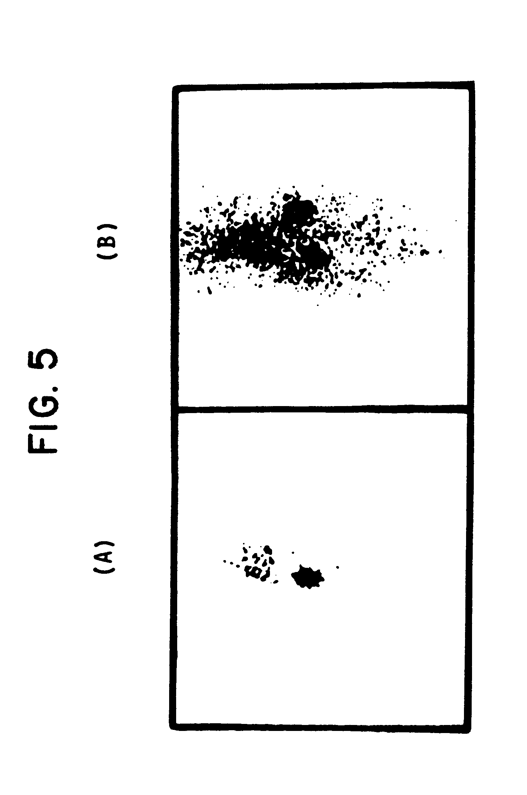

Six to twelve months post-transplantation, the recipient rats were intravenously injected with 10 $\mu g$ $^{111}$In-CYT-015-ADTPA, and imaged as described in Section 8.1. In FIG. 5, images A and B were taken approximately 1 hour and 20 minutes after intravenous injection.

As shown in FIG. 5, image A, the radionuclide localized within the transplanted kidney within approximately 1 hour and 20 minutes after intravenous injection of the labeled antibody-metal ion complex. Minimal accumulation of radioactivity was detected in the animal's kidney, lungs, spleen or liver.

FIG. 5, image B, illustrates the image obtained when the same recipient animal was injected with a commercially available renal imaging agent, $^{99m}$Technetium-DTPA chelate (Mallinkrodt, St. Louis, MO). Clearly, images obtained with $^{99m}$Technetium-DTPA chelate are inferior to those obtained with the antibody-metal ion complexes of the invention.

Both kidneys of the recipient animal were clearly functioning.

## Claims

**Claims for the following Contracting States : BE, CH, DE, FR, GB, IT, LI, LU, NL, SE**

1. An antibody-chelator conjugate, comprising : a compatible chelator attached through a covalent bond to an antibody or antibody fragment and capable of coordinate bonding to a metal ion, in which the covalent bond is between an amine of the compatible chelator and an aldehyde group of an oxidized carbohydrate moiety of the antibody or antibody fragment, said antibody-chelator conjugate having substantially the same immunoreactivity and immunospecificity as the unconjugated antibody or antibody fragment, and wherein the covalent bond is (i) selectively formed at a site located outside the antigen binding region of the antibody or antibody fragment and (ii) selected from an imine, an enamine, hydrazone, oxime, phenylhydrazone, semicarbazone, thiosemicarbazone and a reduced form

thereof.

2. The antibody-chelator conjugate according to claim 1, wherein the antibody fragment is selected from Fab fragments, $(Fab')_2$ fragments and half antibody molecules.

3. The antibody-chelator conjugate according to claim 1 or 2 wherein the antibody or antibody fragment is a monoclonal antibody or monoclonal antibody fragment.

4. The antibody-chelator conjugate according to claims 1 to 3, wherein the compatible chelator is an amine-containing derivative of a chelator selected from diethylenetriamine - pentaacetic acid, ethylenediaminetetraacetic acid, dimercaptosuccinic acid, 2, 3-dimercaptopropanesulfonic acid and metallothionein.

5. The antibody-chelator conjugate according to claims 1 to 4, wherein the compatible chelator is selected from p-aminoaniline-diethylenetriaminepentaacetic acid, hydrazide-diethylenetriaminepentaacetic acid, phenylhydrazide-diethylenetriaminepentaacetic acid, hydroxylamine-diethylenetriaminepentaacetic acid, semicarbazide-diethylenetriaminepentaacetic acid, thiosemicarbazide-diethylenetriaminepentaacetic acid, polyethyleneimine-diethylenetriaminepentaacetic acid, p-phenylenediamine-diethylenetriaminepentaacetic acid, α-N-diethylenetriaminepentaacetic acid-L-lysine, glycyl-tyrosyl-lysine-diethylenetriaminepentaacetic acid, diethylenetriaminepentaacetic acid mono [(4-aminophenyl)methyl] amide and
L-lysine benzyl ester-diethylenetriaminepentaacetic acid.

6. The antibody-chelator conjugate according to claim 1, wherein the compatible chelator is an amine-containing derivative of a cryptate.

7. An antibody-chelator metal ion complex comprising an antibody-chelator conjugate according to claims 1 to 6, said antibody-chelator conjugate coordinatedly bound through said compatible chelator to a metal ion to form said antibody-chelator metal ion complex.

8. The antibody-chelator metal ion complex according to claim 7, substantially free of non-chelated metal ions.

9. The antibody-chelator metal ion complex according to claim 7, wherein the metal ion is a radioisotope.

10. The antibody-chelator metal ion complex according to claim 9, wherein the radioisotope emits beta particles.

11. The antibody-chelator metal ion complex according to claim 10, wherein the radioisotope is selected from $^{46}$scandium, $^{47}$Scandium, $^{48}$Scandium, $^{72}$Gallium and $^{73}$Gallium.

12. The antibody-chelator metal ion complex according to claim 11, wherein the radioisotope emits alpha particles.

13. The antibody-chelator metal ion complex according to claim 12, wherein the radioisotope is selected from $^{211}$Bismith, $^{212}$Bismuth, $^{213}$Bismuth and $^{214}$Bismuth.

14. The antibody-chelator metal ion complex according to claim 7, wherein the radioisotope emits positron particles.

15. The antibody-chelator metal ion complex according to claim 14, wherein the radioisotope is selected from $^{43}$Scandium, $^{44}$Scandium, $^{52}$Iron, $^{55}$Cobalt and $^{68}$Gallium.

16. The antibody-chelator metal ion complex according to claim 7, wherein the metal ion is paramagnetic.

17. The antibody-chelator metal ion complex according to claim 16, wherein the metal ion is selected from $^{54}$Iron, $^{56}$Iron, $^{57}$Iron, $^{58}$Iron, $^{157}$Gadolinium and $^{55}$Manganese.

18. The antibody-chelator metal ion complex according to claims 8 to 17, wherein the antibody or $(Fab')_2$ fragment is a monoclonal antibody or monoclonal antibody $(Fab')_2$ fragment.

19. A composition for therapeutic treatment of cellular disorders, comprising a therapeutically effective amount of an antibody-chelator metal ion complex according to claim 10, said chelator metal ion complex being immunoreactive with and immunospecific for a target site associated with said cellular disorder and substantially non-immunoreactive with and immunospecific for a target site associated with said cellular disorder and non-immunoreactive with and non-immunospecific for tissue not associated with said cellular disorder, and wherein the metal ion emits cytotoxic beta particles.

20. A method for testing for antigen, comprising :
(a) mixing an antibody-chelator metal ion complex according to claims 7 to 18, with a sample suspected of containing a particular antigen, the antibody-chelator metal ion complex being immunoreactive with and immunospecific for said antigen and
(b) detecting any interaction of said antibody-chelator metal ion complex with any antigen in the sample.

**Claims for the following Contracting State : AT**

1. Process for producing an antibody-chelator conjugate, comprising : attaching a compatible chelator through a covalent bond to an antibody or antibody fragment, said chelator being capable of coordinate bonding to a metal ion, in which the covalent bond is between an amine of the compatible chelator and an aldehyde group of an oxidized carbohydrate moiety of the antibody or antibody fragment, said antibody-chelator conjugate having substantially the same immunoreactivity and immunospecificity as the unconjugated antibody or antibody fragment, and wherein the covalent bond is (i) selectively formed at a site located outside the antigen binding region of the antibody or antibody fragment and (ii) selected from an imine, an enamine, hydrazone, oxime, phenylhydrazone, semicarbazone, thiosemicarbazone and a reduced form thereof.

2. The Process according to claim 1, wherein the antibody fragment is selected from Fab fragments, $(Fab')_2$ fragments and half antibody molecules.

3. The Process according to claim 1 or 2, wherein the antibody or antibody fragment is a monoclonal antibody or monoclonal antibody fragment.

4. The Process according to claims 1 to 3, wherein the compatible chelator is an amine-containing derivative of a chelator selected from diethylenetriamine - pentaacetic acid, ethylenediaminetetraacetic acid, dimercaptosuccinic acid, 2, 3-dimercaptopropanesulfonic acid and metallothionein.

5. The Process according to claims 1 to 4, wherein the compatible chelator is selected from p-aminoaniline-diethylenetriaminepentaacetic acid, hydrazide-diethylenetriaminepentaacetic acid, phenylhydrazide-diethylenetriaminepentaacetic acid, hydroxylamine-diethylenetriaminepentaacetic acid, semicarbazide-diethylenetriaminepentaacetic acid, thiosemicarbazide-diethylenetriaminepentaacetic acid, polyethyleneimine-diethylenetriaminepentaacetic acid, p-phenylenediamine-diethylenetriaminepentaacetic acid, $\alpha$-N-diethylenetriaminepentaacetic acid-L-lysine, glycyl-tyrosyl-lysine-diethylenetriaminepentaacetic acid, diethylenetriaminepentaacetic acid mono [(4-aminophenyl)methyl] amide and
L-lysine benzyl ester-diethylenetriaminepentaacetic acid.

6. The Process according to claim 1, wherein the compatible chelator is an amine- containing derivative of a cryptate.

7. A Process for preparing an antibody-chelator metal ion complex comprising coordinately bonding an antibody-chelator conjugate according to claims 1 to 6, through said compatible chelator to a metal ion to form said antibody-chelator metal ion complex

8. The Process according to claim 7, wherein the complex is substantially free of non-chelated metal ions.

9. The Process according to claim 7, wherein the metal ion is a radioisotope.

10. The Process according to claim 9, wherein the radioisotope emits beta particles.

11. The Process according to claim 10, wherein the radioisotope is selected from $^{46}$Scandium, $^{47}$Scandium, $^{72}$Gallium and $^{73}$Gallium

12. The Process according to claim 11, wherein the radioisotope emits alpha particles.

13. The Process according to claim 12, wherein the radioisotope is selected from $^{211}$Bismuth, $^{212}$Bismuth, $^{213}$Bismuth and $^{214}$Bismuth.

14. The Process according to claim 7, wherein the radioisotope emits positron particles.

15. The Process according to claim 14, wherein the radioisotope is selected from $^{43}$Scandium, $^{44}$Scandium, $^{52}$Iron, $^{55}$Cobalt and $^{68}$Gallium.

16. The process according to claim 7, wherein the metal ion is paramagnetic.

17. The Process according to claim 16, wherein the metal ion is selected from $^{54}$Iron, $^{56}$Iron, $^{57}$Iron, $^{58}$Ion, $^{157}$Gadolinium and $^{55}$Manganese.

18. The Process according to claims 8 to 17, wherein the antibody or $(Fab')_2$ fragment is a monoclonal antibody or monoclonal antibody $(Fab')_2$ fragment.

19. A Process for the preparation of a composition for therapeutic treatment of cellular disorders, comprising mixing with a pharmaceutical carrier a therapeutically effective amount of an antibody-chelator metal ion complex obtainable by the process of claim 10, said antibody-chelator metal ion complex being immunoreactive with and immunospecific for a target site associated with said cellular disorder and substantially non-immunoreactive with and immunospecific for a target site associated with said cellular disorder and substantially non-immunoreactive with and non-immunospecific for tissue not associated with said cellular disorder, and wherein the metal ion emits cytotoxic beta particles.

20. A method for testing for antigen, comprising :
(a) mixing an antibody-chelator metal ion complex according to claim 7, with a sample suspected of containing a particular antigen, the antibody-chelator metal ion complex being immunoreactive with and immunospecific for said antigen, and
(b) detecting any interaction of said antibody-chelator metal ion complex with any antigen in the sample.

**Revendications**
**Revendications pour les Etats contractants suivants : BE, CH, DE, FR, GB, IT, LI, LU, NL, SE**

1. Conjugué anticorps-chélateur comprenant : un chélateur compatible fixé par une liaison covalente à un anticorps ou à un fragment d'anticorps et capable de liaison coordonnée à un ion métallique, dans lequel la liaison covalente est entre une amine du chélateur compatible et un groupe aldéhyde d'une portion glucidique oxydée de l'anticorps ou du fragment d'anticorps, ledit conjugué anticorps-chélateur ayant sensiblement la même immunoréactivité et la même immmunospécificité que l'anticorps, ou le fragment d'anticorps, non conjugué et où la liaison covalente est (i) formée sélectivement en un site situé hors de la région de liaison à l'antigène de l'anticorps ou du fragment d'anticorps et (ii) choisie parmi imine, énamine, hydrazone, oxime, phénylhydrazone, semicarbazone, thiosemicarbazone et une forme réduite correspondante.

2. Conjugué anticorps-chélateur selon la revendication 1, où le fragment d'anticorps est choisi parmi les fragments Fab, $(Fab')_2$ et les demi-molécules d'anticorps.

3. Conjugué anticorps-chélateur selon la revendication 1 ou 2, où l'anticorps ou fragment d'anticorps est un anticorps monoclonal ou un fragment d'anticorps monoclonal.

4. Conjugué anticorps-chélateur selon les revendications 1 à 3, où le chélateur compatible est un dérivé contenant une amine d'un chélateur choisi parmi l'acide diéthylènetriaminepentaacétique, l'acide éthylènediaminetétraacétique, l'acide dimercaptosuccinique, l'acide 2,3-dimercaptopropanesulfonique et une métallothionéine.

5. Conjugué anticorps-chélateur selon les revendications 1 à 4, où le chélateur compatible est choisi parmi l'acide p-aminoaniline-diéthylènetriaminepentaacétique, l'acide hydrazide-diéthylènetriaminepentaacétique, l'acide phénylhydrazide-diéthylènetriaminepentaacétique l'acide hydroxylamine-diéthylènetriaminepentaacétique, l'acide semicarbazide-diéthylènetriaminepentaacétique, l'acide thiosemicarbazide-diéthylènetriaminepentaacétique, l'acide polyéthylène-imine-diéthylènetriaminepentaacétique, l'acide p-phénylènediamine-diéthylènetriaminepentaacétique l'acide α-N-diéthylènetriaminepentaacétique-L-lysine, l'acide glycyl-tyrosyl-lysine-diethylènetriaminepentaacétique, le mono[(4-aminophényl)méthyl]amide de l'acide diéthylènetriaminepentaacétique et
l'ester benzylique de L-lysine-acide diéthylènetriaminepentaacétique.

6. Conjugué anticorps-chélateur selon la revendication 1, dans lequel le chélateur compatible est un dérivé contenant une amine d'un cryptate.

7. Complexe anticorps-chélateur-ion métallique comprenant un conjugué anticorps-chélateur selon les revendications 1 à 6, ledit conjugué anticorps-chélateur étant lié par coordinance, via ledit chélateur compatible, à un ion métallique pour former ledit complexe anticorps-chélateur-ion métallique.

8. Complexe anticorps-chélateur-ion métallique selon la revendication 7 sensiblement dépourvu d'ions métalliques non chélatés.

9. Complexe anticorps-chélateur-ion métallique selon la revendication 7, dans lequel l'ion métallique est un radio-isotope.

10. Complexe anticorps-chélateur-ion métallique selon la revendication 9, dont le radio-isotope émet des particules $\beta$.

11. Complexe anticorps-chélateur-ion métallique selon la revendication 10, dont le radio-isotope est choisi parmi le scandium 46, le scandium 47, le scandium 48, le gallium 72 et le gallium 73.

12. Complexe anticorps-chélateur-ion métallique selon la revendication 11, dont le radio-isotope émet des particules $\alpha$.

13. Complexe anticorps-chélateur-ion métallique selon la revendication 12, dont le radio-isotope est choisi parmi le bismuth 211, le bismuth 212, le bismuth 213 et le bismuth 214.

14. Complexe anticorps-chélateur-ion métallique selon la revendication 7, dont le radio-isotope émet des positons.

15. Complexe anticorps-chélateur-ion métallique selon la revendication 14, dont le radio-isotope est choisi parmi le scandium 43, le scandium 44, le fer 52, le cobalt 55 et le gallium 68.

16. Complexe anticorps-chélateur-ion métallique selon la revendication 7, dont l'ion métallique est paramagnétique.

17. Complexe anticorps-chélateur-ion métallique selon la revendication 16, dont l'ion métallique est choisi parmi le fer 54, le fer 56, le fer 57, le fer 58, le gadolinium 157 et le manganèse 55.

18. Complexe anticorps-chélateur-ion métallique selon les revendications 8 à 17, dans lequel l'anticorps ou le fragment $(Fab')_2$ est un anticorps monoclonal ou un fragment $(Fab')_2$ d'anticorps monoclonal.

19. Composition pour le traitement thérapeutique de troubles cellulaires, comprenant une quantité thérapeutique efficace d'un complexe anticorps-chélateur-ion métallique selon la revendication 10, ledit complexe chélateur-ion métallique étant immunoréactif et immunospécifique relativement à un site cible

associé audit trouble cellulaire et sensiblement non immunoréactif et immunospécifique relativement à un site cible associé audit trouble cellulaire et non immunoréactif et non immunospécifique relativement à un tissu non associé audit trouble cellulaire et dont l'ion métallique émet des particules $\beta$ cytotoxiques.

**20.** Procédé d'essai d'un antigène comprenant :
(a) le mélange d'un complexe anticorps-chélateur-ion métallique selon les revendications 7 à 18 avec un échantillon suspect de contenir un antigène particulier, le complexe anticorps-chélateur-ion métallique étant immunoréactif et immunospécifique relativement audit antigène et
(b) la détection de toute interaction dudit complexe anticorps-chélateur-ion métallique avec un antigène dans l'échantillon.

**Revendications pour l'Etat contractant suivant : AT**

**1.** Procédé pour produire un conjugué anticorps-chélateur comprenant : la fixation d'un chélateur compatible par une liaison covalente à un anticorps ou fragment d'anticorps, ledit chélateur étant capable de liaison coordonnée à un ion métallique, dans lequel la liaison covalente se situe entre une amine du chélateur compatible et un groupe aldéhyde d'une portion glucidique oxydée de l'anticorps ou du fragment d'anticorps, ledit conjugué anticorps-chélateur ayant sensiblement la même immunoréactivité et la même immunospécificité que l'anticorps, ou le fragment d'anticorps, non conjugué, et dans lequel la liaison covalente est (i) formée sélectivement en un site situé hors de la région de liaison à l'antigène de l'anticorps ou du fragment d'anticorps et (ii) choisie parmi imine, énamine, hydrazone, oxime, phénylhydrazone, semicarbazone, thiosemicarbazone et une forme réduite correspondante.

**2.** Procédé selon la revendication 1, dans lequel le fragment d'anticorps est choisi parmi les fragments Fab, $(Fab')_2$ et les demi-molécules d'anticorps.

**3.** Procédé selon la revendication 1 ou 2, dans lequel l'anticorps ou fragment d'anticorps est un anticorps monoclonal ou un fragment d'anticorps monoclonal.

**4.** Procédé selon les revendications 1 à 3, dans lequel le chélateur compatible est un dérivé contenant une amine d'un chélateur choisi parmi l'acide diéthylènetriaminepentaacétique, l'acide éthylènediaminetétraacétique, l'acide dimercaptosuccinique, l'acide 2,3-dimercaptopropanesulfonique et une métallothionéine.

**5.** Procédé selon les revendications 1 à 4, dans lequel le chélateur compatible est choisi parmi :
l'acide p-aminoaniline-diéthylènetriaminepentaacétique, l'acide hydrazide-diéthylènetriaminepentaacétique, l'acide phénylhydrazide-diéthylènetriaminepentaacétique, l'acide hydroxylamine-diéthylènetriaminepentaacétique, l'acide semicarbazide-diéthylènetriaminepentaacétique, l'acide thiosemicarbazide-diéthylènetriaminepentaacétique, l'acide polyéthylène-imine-diéthylènetriaminepentaacétique, l'acide p-phénylènediamine-diéthylènetriaminepentaacétique, l'acide $\alpha$-N-diéthylènetriaminepentaacétique-L-lysine, l'acide glycyl-tyrosyl-lysine-diéthylènetriaminepentaacétique, le mono[(4-aminophényl)méthyl]amide de l'acide diéthylènetriaminepentaacétique et
l'ester benzylique de L-lysine-acide diéthylènetriaminepentaacétique.

**6.** Procédé selon la revendication 1, dans lequel le chélateur compatible est un dérivé contenant une amine d'un cryptate.

**7.** Procédé pour préparer un complexe anticorps-chélateur-ion métallique comprenant la liaison coordonnée d'un conjugué anticorps-chélateur selon les revendications 1 à 6, via ledit chélateur compatible, à un ion métallique pour former ledit complexe anticorps-chélateur-ion métallique.

**8.** Procédé selon la revendication 7, dans lequel le complexe est sensiblement dépourvu d'ions métalliques non chélatés.

**9.** Procédé selon la revendication 7, dans lequel l'ion métallique est un radio-isotope.

**10.** Procédé selon la revendication 9, dans lequel le radio-isotope émet des particules $\beta$.

**11.** Procédé selon la revendication 10, dans lequel le radio-isotope est choisi parmi le scandium 46, le scandium 47, le scandium 48, le gallium 72 et le gallium 73.

**12.** Procédé selon la revendication 11, dans lequel le radio-isotope émet des particules $\alpha$.

**13.** Procédé selon la revendication 12, dans lequel le radio-isotope est choisi parmi le bismuth 211, le bismuth 212, le bismuth 213 et le bismuth 214.

**14.** Procédé selon la revendication 7, dans lequel le radio-isotope émet des positons.

**15.** Procédé selon la revendication 14, dans lequel le radio-isotope est choisi parmi le scandium 43, le scandium 44, le fer 52, le cobalt 55 et le gallium 68.

**16.** Procédé selon la revendication 7, dans lequel l'ion métallique est paramagnétique.

**17.** Procédé selon la revendication 16, dans lequel l'ion métallique est choisi parmi le fer 54, le fer 56, le fer 57, le fer 58, le gadolinium 157 et le manganèse 55.

**18.** Procédé selon les revendications 8 à 17, dans lequel l'anticorps ou le fragment $(Fab')_2$ est un anticorps monoclonal ou un fragment $(Fab')_2$ d'anticorps monoclonal.

**19.** Procédé pour la préparation d'une composition pour le traitement thérapeutique de troubles cellulaires, comprenant le mélange avec un véhicule pharmaceutique d'une quantité thérapeutique efficace d'un complexe anticorps-chélateur-ion métallique pouvant être obtenu selon le procédé de la revendication 10, ledit complexe anticorps-chélateur-ion métallique étant immunoréactif et immunospécifique relativement à un site cible associé audit trouble cellulaire et sensiblement non immunoréactif et immunospécifique relativement à un site cible associé audit trouble cellulaire et sensiblement non immunoréactif et non immunospécifique relativement à un tissu non associé audit trouble cellulaire et dont l'ion métallique émet des particules $\beta$ cytotoxiques.

**20.** Procédé d'essai d'un antigène comprenant :
(a) le mélange d'un complexe anticorps-chélateur-ion métallique selon la revendication 7 avec un échantillon suspect de contenir un antigène particulier, le complexe anticorps-chélateur-ion métallique étant immunoréactif et immunospécifique relativement audit antigène et
(b) la détection de toute interaction dudit complexe anticorps-chélateur-ion métallique avec un antigène dans l'échantillon.

**Patentansprüche**
**Patentansprüche für folgende Vertragsstaaten : BE, CH, DE, FR, GB, IT, LI, LU, NL, SE**

**1.** Antikörperchelatverbindung, die folgendes umfaßt: Ein kompatibles Chelat, verbunden mittels einer Kovalenzbindung mit einem Antikörper oder Antikörperfragment und in der Lage, eine koordinierte Bindung an ein Metall-Ion einzugehen, in dem die Kovalenzbindung zwischen einem Amin des kompatiblen Chelats und einer Aldehydgruppe eines oxidierten Kohlenhydratanteils des Antikörpers oder Antikörperfragments besteht, wobei besagte Antikörperchelatverbindung im wesentlichen dieselbe Immunisierungsreaktivität und Immunisierungsspezifizität wie der nicht-gekoppelte Antikörper oder das nicht-gekoppelte Antikörperfragment besitzt, und worin die Kovalenzbindung (i) selektiv an einer Stelle gebildet wird, die sich außerhalb des antigenen Bindungsbereichs des Antikörpers oder Antikörperfragments befindet, und (ii) aus einem Imin, Enamin, Hydrazon, Oxim, Phenylhydrazon, Semikarbazon, Thiosemikarbazon und einer reduzierten Form derselben selektiert wird.

**2.** Antikörperchelatverbindung nach Anspruch 1, worin das Antikörperfragment aus Fab-Fragmenten, $(Fab')_2$-Fragmenten und Halbantikörpermolekülen selektiert wird.

**3.** Antikörperchelatverbindung nach den Ansprüchen 1 oder 2, worin der Antikörper oder das Antikörperfragment ein monoklonaler Antikörper oder ein monoklonales Antikörperfragment ist.

**4.** Antikörperchelatverbindung nach den Ansprüchen 1 bis 3, worin das kompatible Chelat ein Amin

enthaltendes Derivat eines Chelats ist, das aus diäthylentriamin-pentaazetischer Säure, äthylen-diamintetraazetischer Säure, dimercaptosuccinischer Säure, 2,3-dimercaptopropansulfonischer Säure und Matallothionein selektiert wird.

**5.** Antikörperchelatverbindung nach den Ansprüchen 1 bis 4, worin das kompatible Chelat aus P-aminoanilin-diäthylentriamin-pentaazetischer Säure, hydrazid-diäthylentriamin-pentaazetischer Säure, Phenylhydrazid-diäthylentriamin-pentaazetischer Säure, hydroxylamin-diäthylentriamin-pentaazetischer Säure, Semicarbazid-diäthylentriamin-pentaazetischer Säure, thiosemicarbazid-diäthylentriamin-pentaazetischer Säure, polyäthylenamin-diäthylentriamin-pentaazetischer Säure, p-phenylendiamin-diäthylentriamin-pentaazetischer Säure, α-N-diäthylentriamin-pentaazetischer Säure-L-Lysin, glycyl-tyrosyl-lysin-diäthylentriaminpentaazetischer Säure, diäthylentriamin-pentaazetischem Säuremono, [(4-Aminophenyl)methyl] -Amid **und**
L-lysin-benzyl-ester-diäthylentriamin-pentaazetischer Säure selektiert wird.

**6.** Antikörperchelatverbindung nach Anspruch 1, worin das kompatible Chelat ein Amin enthaltendes Derivat eines Kryptats ist.

**7.** Antikörperchelatmetall-Ionkomplex, der eine Antikörperchelatverbindung nach den Ansprüchen 1 bis 6 umfasst, wobei besagte Antikörperchelatverbindung mittels besagtem kompatiblem Chelat mit einem Metall-Ion zwecks Bildung des besagten Antikörperchelatmetall-Ionkomplexes koordiniert verbunden ist.

**8.** Antikörperchelatmetall-Ionkomplex nach Anspruch 7, im wesentlichen frei von nicht-chelatierten Metall-Ionen.

**9.** Antikörperchelatmetall-Ionkomplex nach Anspruch 7, worin das Metall-Ion ein Radioisotop ist.

**10.** Antikörperchelatmetall-Ionkomplex nach Anspruch 9, worin das Radioisotop Betapartikel ausstrahlt.

**11.** Antikörperchelatmetall-Ionkomplex nach Anspruch 10, worin das Radioisotop aus $^{46}$Scandium, $^{47}$Scandium, $^{48}$Scandium, $^{72}$Gallium und$^{73}$Gallium selektiert wird.

**12.** Antikörperchelatmetall-Ionkomplex nach Anspruch 11, worin das Radioisotop Alphapartikel ausstrahlt.

**13.** Antikörperchelatmetall-Ionkomplex nach Anspruch 12, worin das Radioisotop aus $^{211}$Wismut, $^{212}$Wismut, $^{213}$Wismut und $^{214}$Wismut selektiert wird.

**14.** Antikörperchelatmetall-Ionkomplex nach Anspruch 7, worin das Radioisotop Positronpartikel ausstrahlt.

**15.** Antikörperchelatmetall-Ionkomplex nach Anspruch 14, worin das Radioisotop aus $^{43}$Scandium, $^{44}$Scandium, $^{52}$Eisen, $^{55}$Kobalt und$^{68}$Gallium selektiert wird.

**16.** Antikörperchelatmetall-Ionkomplex nach Anspruch 7, worin das Metall-Ion paramagnetisch ist.

**17.** Antikörperchelatmetall-Ionkomplex nach Anspruch 16, worin das Metall-Ion aus $^{54}$Eisen, $^{56}$Eisen, $^{57}$Eisen, $^{58}$Eisen, $^{157}$Gadolinium und$^{55}$Mangan selektiert wird.

**18.** Antikörperchelatmetall-Ionkomplex nach den Ansprüchen 8 bis 17, worin der Antikörper oder das $(Fab')_2$-Fragment ein monoklonaler Antikörper oder ein monoklonales Antikörper-$(Fab')_2$-Fragment ist.

**19.** Verbindung für die therapeutische Behandlung von Zellerkrankungen, welche eine therapeutisch effektive Menge eines Antikörperchelatmetall-Ionkomplexes nach Anspruch 10 umfaßt, wobei der besagte Chelatmetall-Ionkomplex immunreaktiv gegen und immunspezifisch für einen Zielort ist, der mit besagter Zellerkrankung zusammenhängt, und im wesentlichen nicht immunreaktiv gegen und immunspezifisch für einen Zielort ist, der mit besagter Zellerkrankung zusammenhängt, und nicht immunreaktiv gegen und nicht immunspezifisch für Gewebe ist, das mit besagter Zellerkrankung nicht zusammenhängt, und worin das Metall-Ion zytotoxische Betapartikel ausstrahlt.

**20.** Verfahren zur Prüfung von Antigen, das folgendes umfaßt:

a) das Vermischen eines Antikörperchelatmetall-Ionkomplexes nach den Ansprüchen 7 bis 18 mit einer Probe, bei der das Vorhandensein eines speziellen Antigens vermutet wird, wobei der Antikörperchelatmetall-Ionkomplex immunreaktiv gegen und immunspezifisch für besagtes Antigen ist **und**

b) die Feststellung einer Wechselwirkung von besagtem Antikörperchelatmetall-Ionkomplex mit jeglichem Antigen in der Probe.

**Patentansprüche für folgenden Vertragsstaat : AT**

1. Verfahren zur Herstellung einer Antikörperchelatverbindung, das folgendes umfasst: Die Verbindung eines kompatiblen Chelats mittels Kovalenzbindung mit einem Antikörper oder Antikörperfragment, wobei besagtes Chelat in der Lage ist, eine koordinierte Bindung an ein Metall-Ion einzugehen, in dem die Kovalenzbindung zwischen einem Amin des kompatiblen Chelats und einer Aldehydgruppe eines oxidierten Kohlenhydratanteils des Antikörpers oder Antikörperfragments besteht, und wobei die besagte Antikörperchelatverbindung im wesentlichen dieselbe Immunreaktivität und Immunspezifizität wie der nicht-gekoppelte Antikörper oder das nicht-gekoppelte Antikörperfragment besitzt, und worin die Kovalenzbindung (i) selektiv an einer Stelle gebildet wird, die sich ausserhalb des antigenen Bindungsbereichs des Antikörpers oder Antikörperfragments befindet, und (ii) aus einem Imin, Enamin, Hydrazon, Oxim, Phenylhydrazon, Semicarbazon, Thiosemicarbazon und einer reduzierten Form derselben selektiert wird.

2. Verfahren nach Anspruch 1, worin das Antikörperfragment aus Fab-Fragmenten, $(Fab')_2$-Fragmenten und Halbantikörpermolekülen selektiert wird.

3. Verfahren nach den Ansprüchen 1 oder 2, worin der Antikörper oder das Antikörperfragment ein monoklonaler Antikörper oder ein monoklonales Antikörperfragment ist.

4. Verfahren nach den Ansprüchen 1 bis 3, worin das kompatible Chelat ein Amin enthaltendes Derivat eines Chelats ist, das aus diäthylentriamin-pentaazetischer Säure, äthylendiamintetraazetischer Säure, dimercaptosuccinischer Säure, 2,3-dimercaptopropansulfonischer Säure und Metallothionein selektiert wird.

5. Verfahren nach den Ansprüchen 1 bis 4, worin das kompatible Chelat aus p-aminoanilin-diäthylentriamin-pentaazetischer Säure, hydrazid-diäthylentriamin-pentaazetischer Säure, phenylhydrazid-diäthylentriamin-pentaazetischer Säure, hydroxylamin-diäthylentriamin-pentaazetischer Säure, semicarbazid-diäthylentriamin-pentaazetischer Säure, thiosemicarbazid-diäthylentriamin-pentaazetischer Säure, polyäthylenimin-diäthylentriamin-pentaazetischer Säure, p-phenylendiamin-diäthylentriamin-pentaazetischer Säure, $\alpha$-N-diäthylentriamin-pentaazetischer Säure-L-Lysin,glycyltriosyl-lysin-diäthylentriamin-pentaazetischer Säure, diäthylentriamin-pentaazetischem Säuremono, [(4-Aminophenyl)methyl]-Amid **und**
L-lysin-benzyl-ester-diäthylentriamin-pentaazetischer Säure selektiert wird.

6. Verfahren nach Anspruch 4, worin das kompatible Chelat ein Amin enthaltendes Derivat eines Kryptats ist.

7. Verfahren zur Bereitung eines Antikörperchelatmetall-Ionkomplexes, das die koordinierte Verbindung einer Antikörperchelatverbindung nach den Ansprüchen 1 bis 6 mittels besagtem kompatiblen Chelat mit einem Metall-Ion zwecks Bildung des besagten Antikörperchelatmetall-Ionkomplexes umfasst.

8. Verfahren nach Anspruch 7, worin der Komplex im wesentlichen frei von nicht-chelatierten Metall-Ionen ist.

9. Verfahren nach Anspruch 7, worin das Metall-Ion ein Radioisotop ist.

10. Verfahren nach Anspruch 9, worin das Radioisotop Betapartikel ausstrahlt.

11. Verfahren nach Anspruch 10, worin das Radioisotop aus $^{46}$Scandium, $^{47}$Scandium, $^{72}$Gallium und $^{73}$Gallium selektiert wird.

**12.** Verfahren nach Anspruch 11, worin das Radioisotop Alphapartikel ausstrahlt.

**13.** Verfahren nach Anspruch 12, worin das Radioisotop aus $^{211}$Wismut, $^{212}$Wismut, $^{213}$Wismut und $^{214}$-Wismut selektiert wird.

**14.** Verfahren nach Anspruch 7, worin das Radioisotop Positronpartikel ausstrahlt.

**15.** Verfahren nach Anspruch 14, worin das Radioisotop aus $^{43}$Scandium, $^{44}$Scandium, $^{52}$ Eisen, $^{55}$Kobalt und $^{68}$ Gallium selektiert wird.

**16.** Verfahren nach Anspruch 7, worin das Metall-Ion paramagnetisch ist.

**17.** Verfahren nach Anspruch 16, worin das Metall-Ion aus $^{54}$Eisen, $^{56}$Eisen, $^{57}$Eisen, $^{58}$Eisen, $^{157}$Gadolinium und $^{55}$Mangan selektiert wird.

**18.** Verfahren nach den Ansprüchen 8 bis 17, worin der Antikörper oder das $(Fab')_2$-Fragment ein monoklonaler Antikörper oder ein monoklonales Antikörper-$(Fab')_2$-Fragment ist.

**19.** Verfahren zur Bereitung einer Verbindung, für die therapeutische Behandlung von Zellerkrankungen, welche die Vermischung einer therapeutisch effektiven Menge eines Antikörperchelatmetall-Ionkomplexes, der durch das Verfahren nach Anspruch 10 erzielbar ist, mit einem pharmazeutischen Träger umfasst, wobei besagter Antikörperchelatmetall-Ionkomplex immunreaktiv gegen und immunspezifisch für einen Zielort ist, der mit besagter Zellerkrankung zusammenhängt, und im wesentlichen nicht immunreaktiv gegen und immunspezifisch für einen Zielort ist, der mit besagter Zellerkrankung zusammenhängt, und im wesentlichen nicht immunreaktiv gegen und nicht immunspezifisch für Gewebe ist, das mit besagter Zellerkrankung nicht zusammenhängt, und worin das Metall-Ion zytotoxische Betapartikel ausstrahlt.

**20.** Verfahren zur Prüfung von Antigen, das folgendes umfasst:
a) das Vermischen eines Antikörperchelatmetall-Ionkomplexes nach Anspruch 7 mit einer Probe, bei der das Vorhandensein eines speziellen Antigens vermutet wird, wobei der Antikörperchelatmetall-Ionkomplex immunreaktiv gegen und immunspezifisch für besagtes Antigen ist, **und**
b) die Feststellung einer Wechselwirkung von besagtem Antikörperchelatmetall-Ionkomplex mit jeglichem Antigen in der Probe.

FIG. 1

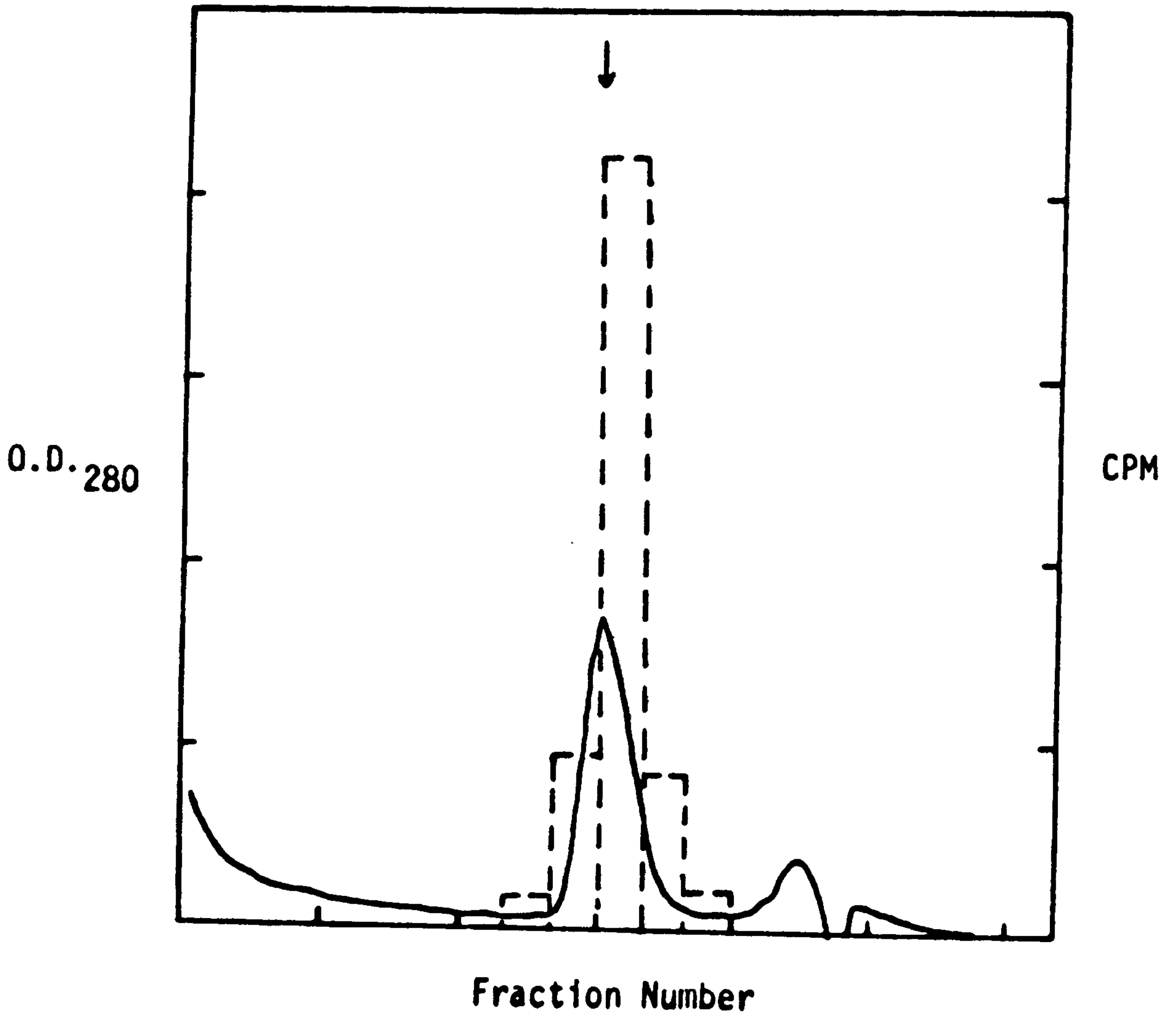

FIG. 2

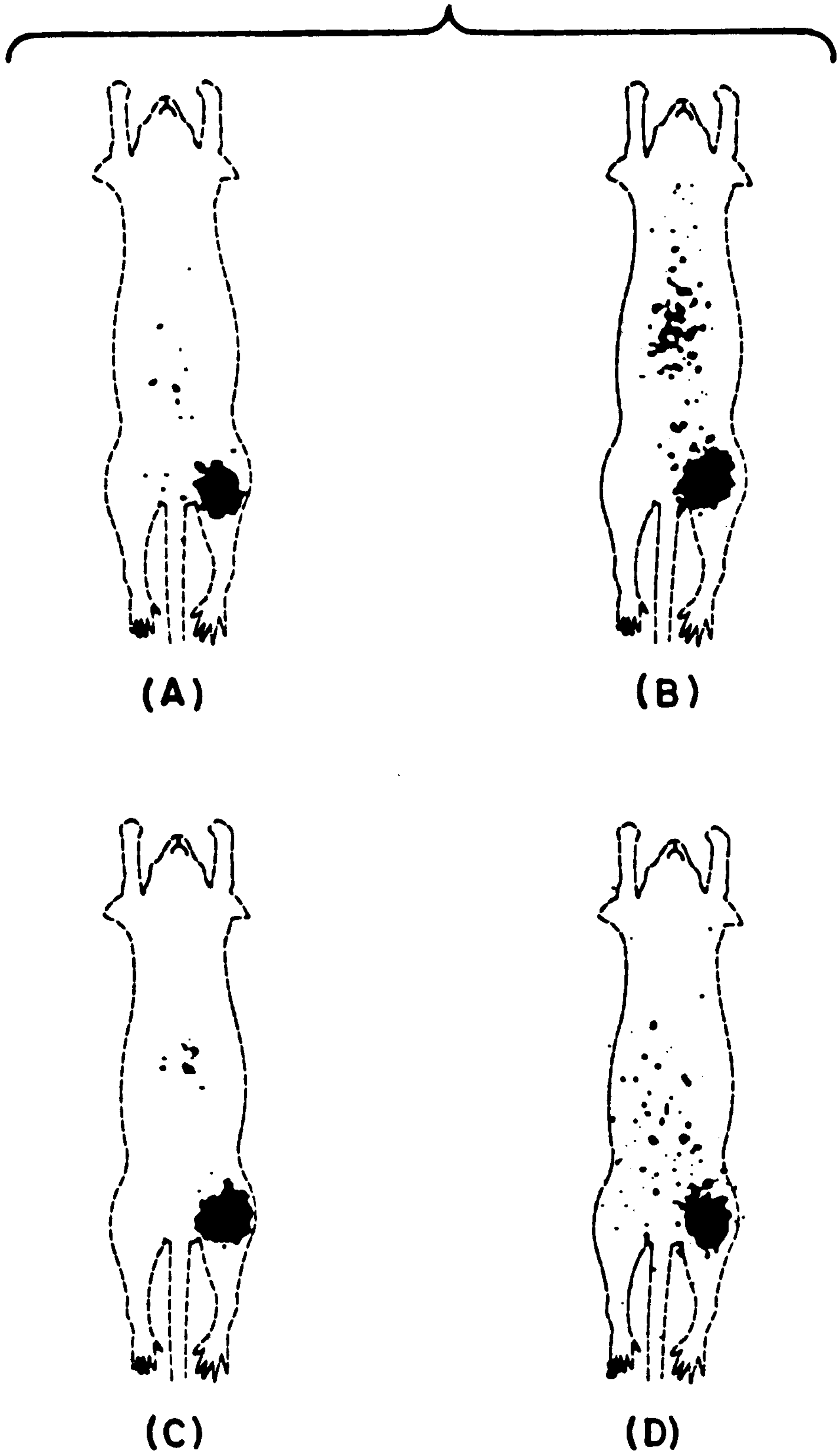

FIG. 3

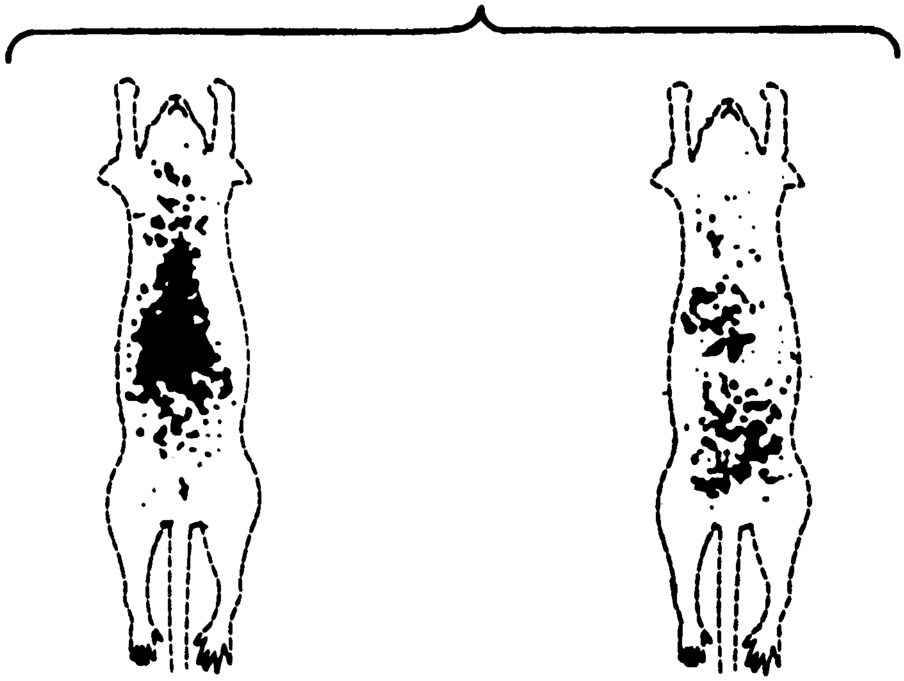

(E) (F)

FIG. 4

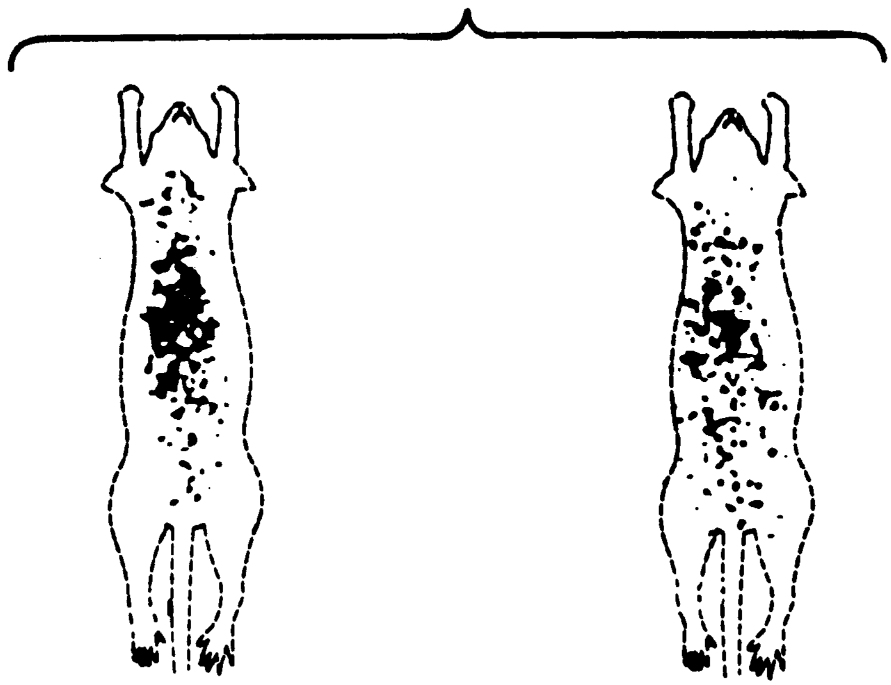

(G) (H)

FIG. 5

(A) (B)